# EUROPEAN PATENT APPLICATION

(11) **EP 2 957 210 A1**
(43) Date of publication of application: **23.12.2015**
(21) Application number: 14751430.1
(22) Date of filing: 24.01.2014
(51) Int. Cl.: A61B 1/00

(54) **ACTIVATION DEVICE**

(30) Priority: 14.02.2013 JP 2013027051
(71) Applicant: OLYMPUS CORPORATION, Shibuya-ku Tokyo 151-0072 (JP)
(72) Inventor: CHIBA, Atsushi, Tokyo 151-0072 (JP); SEGAWA, Hidetake, Tokyo 151-0072 (JP)
(74) Representative: Gunzelmann, Rainer
(86) International application number: PCT/JP2014/051571
(87) International publication number: WO 2014/125908

(57) **Abstract**

A small-sized activation device is provided that can reduce power consumption and easily, reliably, and efficiently activate a capsule medical device while the capsule medical device is housed in a container. An activation device 110 activates a capsule endoscope 10 that has a receiving coil 18a inside and is activated when voltage not less than a specified value or current not less than a specified value is generated in the receiving coil 18a. The activation device includes: a case 111; a transmitting coil 114 provided in the case 111 and configured to generate a magnetic field when current flows through the transmitting coil; and a guide display portion 112 for guiding to a position that is located outside the case 111 and that is a position of the capsule endoscope 10 where the capsule endoscope 10 can be activated based on the magnetic field generated by the transmitting coil 114.

## Description

### Field

The present invention relates to an activation device for switching a power supply of a capsule medical device, which is configured to be inserted into a subject to capture an image in the subject, from an off state to an on state.

### Background

In recent years, development of a capsule medical device that is inserted into a subject and performs a specified operation is progressed in the medical field. In particular, in the endoscope field, a capsule endoscope that has an imaging function and a wireless communication function is practically used. From when the capsule endoscope is inserted into a subject to when the capsule endoscope is discharged from the subject, the capsule endoscope captures images while moving in the subject and sequentially wirelessly transmits in-vivo image data acquired thereby to a receiving device provided outside the subject. In an examination using such a capsule endoscope, a doctor can observe the inside of the subject and perform diagnosis by causing a display device to display in-vivo images based on the data accumulated in the receiving device.

The power of the capsule medical device is normally supplied from a built-in battery. Therefore, before starting an examination by the capsule medical device, an operation to turn on an activation switch provided in the capsule medical device and start supply of power from the battery is performed.

As an activation method of the capsule medical device, a method is known which activates the capsule medical device by providing a reed switch in the capsule medical device and bringing a permanent magnet close to the capsule medical device (for example, see Patent Literature 1). Further, a method is also known which activates the capsule medical device by providing a coil in both the activation device and the capsule medical device and generating an induced voltage and/or an induced current in the coil in the capsule medical device (receiving coil) by causing the capsule medical device to pass through an opening of the coil in the activation device (transmitting coil) (for example, see Patent Literature 2).

### Citation List

### Patent Literature

Patent Literature 1: JP 2006-94933 A
Patent Literature 2: JP 2009-516562 W

### Summary

### Technical Problem

The capsule medical device is housed in a sterilized container until immediately before the capsule medical device is introduced into the subject. It is therefore preferred that the capsule medical device be activated while the capsule medical device is housed in the container. However, if the method disclosed in Patent Literature 2 is employed to activate the capsule while the capsule is housed in the container, a diameter of the opening of the transmitting coil has to be large enough for allowing the container to pass through the opening, which results in a large-sized activation device. In addition, a high starting voltage is required depending on the diameter of the opening of the transmitting coil, which increases power consumption. Further, the capsule medical device is inserted into and removed from the opening of the transmitting coil, which makes the activation operation of the capsule medical device cumbersome and complicated.

Here, in order to generate an induced voltage or an induced current in the receiving coil, the receiving coil does not always have to pass through the opening of the transmitting coil. If a positional relationship between the receiving coil and the transmitting coil is set appropriately, it is possible to obtain a necessary induced voltage or induced current. In this case, however, because transmitting efficiency is lower than that of the method in which the receiving coil passes through the opening, the necessary induced voltage or induced current cannot be obtained if the positional relationship between the receiving coil and the transmitting coil is not appropriate. This may disable the activation of the capsule medical device.

The present invention has been made in view of the foregoing, and an object of the invention is to provide a small-sized activation device that is able to easily, reliably, and efficiently activate a capsule medical device while the capsule medical device is housed in a container, as well as to reduce power consumption.

### Solution to Problem

In order to solve the above-described problems and achieve the object, an activation device according to the invention activates a capsule medical device that has a first coil inside and is activated when voltage greater than or equal to a specified value or current greater than or equal to a specified value is generated in the first coil. The activation device includes: a case; a second coil that is provided in the case and is configured to generate a magnetic field when current flows through the second coil; and a guide portion for guiding to a position that is located outside the case and that is a position of the capsule medical device where the capsule medical device can be activated based on the magnetic field generated by the second coil.

In the above-described activation device, when the second coil generates a magnetic field with a predefined pattern, and voltage or current having a predefined pattern is generated in the first coil, the capsule medical device is activated.

In the above-described activation device, the capsule medical device is housed at a specified position in a specified posture in a container having a specified shape. The case has a placement surface on which the container is configured to be placed. The guide portion is configured to guide to a specific position on the placement surface where the capsule medical device can be activated when the container is placed on the placement surface.

In the above-described activation device, the guide portion is display indicating the specific position.

In the above-described activation device, the guide portion is a concave portion or a convex portion provided at the specific position.

In the above-described activation device, the guide portion is a member provided adjacent to the specific position and having an abutting surface to abut against a part of the container.

The above-described activation device further includes a detection unit configured to detect that the container is placed at the specific position and to output a detection signal, and a control unit configured to cause current to be applied to the second coil when the detection unit outputs the detection signal.

In the above-described activation device, the capsule medical device is housed at a specified position in a specified posture in a container having a specified shape. The case has a placement surface on which the container is configured to be placed. The guide portion includes: a detection unit configured to detect a position of the capsule medical device placed on the placement surface and to output a signal indicating the position; and a guidance unit configured to guide the container to a specific position on the placement surface where the capsule medical device can be activated when the container is placed on the placement surface, based on the signal.

In the above-described activation device, the guidance unit includes a light-emitting element provided on the placement surface.

In the above-described activation device, the container is provided with a portion to be detected that can be detected by the detection unit, and the detection unit outputs the signal when detecting the portion to be detected.

The above-described activation device further includes a control unit configured to cause current to be applied to the second coil based on the signal outputted by the detection unit.

In the above-described activation device, the guide portion includes: a signal generator configured to generate a signal to apply a test current to the second coil; a magnetic field detection unit configured to detect a change in magnetic field outside the case; and a control unit configured to cause the current applied to the second coil to increase when the magnetic field detection unit detects the change in magnetic field greater than or equal to a specified value.

In the above-described activation device, the guide portion further includes an indication display unit configured to perform a specified display according to the change in magnetic field detected by the magnetic field detection unit.

In the above-described activation device, the indication display unit is at least one of a light-emitting element, an indicator, and a display panel, which are provided on the placement surface.

### Advantageous Effects of Invention

According to the invention, an activation device is provided with a guide portion that indicates a position which is located outside a case including a second coil and which is a position of a capsule medical device where the capsule medical device can be activated based on a magnetic field generated by the second coil, so that it is possible to easily, reliably, and efficiently activate the capsule medical device while the capsule medical device is housed in a container, as well as to realize small-sized activation device that can reduce power consumption.

### Brief Description of Drawings

FIG. 1 is a perspective view illustrating an appearance of an activation system including an activation device according to Embodiment 1-1 of the present invention.
FIG. 2 is a schematic diagram illustrating a configuration example of the activation system illustrated in FIG. 1.
FIG. 3 is a schematic diagram illustrating a structure of a capsule endoscope illustrated in FIG. 2.
FIG. 4 is a block diagram illustrating an internal configuration of the capsule endoscope illustrated in FIG. 2.
FIG. 5 is a circuit diagram illustrating a configuration example of a switch unit illustrated in FIG. 4.
FIG. 6 is a circuit diagram illustrating an internal configuration of the activation device illustrated in FIG. 2.
FIG. 7 is a diagram illustrating an example of a signal pattern of a magnetic field that activates the capsule endoscope.
FIG. 8 is a perspective view illustrating an appearance of an activation device according to Embodiment 1-2 of the present invention.
FIG. 9 is a schematic diagram illustrating a configuration example of the activation system illustrated in FIG. 8.
FIG. 10 is a schematic diagram explaining a condition given in order to more reliably activate the capsule endoscope illustrated in FIG. 9.
FIG. 11 is a cross-sectional view illustrating a configuration example of an activation system including an activation device according to Embodiment 1-3 of the present invention.
FIG. 12 is a perspective view illustrating an appearance of an activation system including an activation device according to Embodiment 1-4 of the present invention.
FIG. 13 is a schematic diagram illustrating a configuration example of the activation system illustrated in FIG. 12.
FIG. 14 is a schematic diagram illustrating a configuration example of an activation system including an activation device according to Modified Example 1-1.
FIG. 15 is a schematic diagram illustrating a configuration example of an activation system including an activation device according to Embodiment 2-1 of the present invention.
FIG. 16 is a perspective view illustrating a configuration example of an activation system including an activation device according to Embodiment 2-2 of the present invention.
FIG. 17 is a perspective view illustrating a configuration example of an activation system including an activation device according to Embodiment 2-3 of the present invention.
FIG. 18 is a schematic diagram illustrating a configuration example of an activation system including an activation device according to Embodiment 2-4 of the present invention.
FIG. 19 is a top view illustrating a placement surface of the activation device illustrated in FIG. 18.
FIG. 20 is a schematic diagram illustrating a configuration example of an activation system including an activation device according to Embodiment 3-1 of the present invention.
FIG. 21 is a flowchart illustrating an operation of the activation system illustrated in FIG. 20.
FIG. 22 is a schematic diagram illustrating a configuration example of an activation system including an activation device according to Modified Example 3-1-2.
FIG. 23 is a schematic diagram illustrating a configuration example of an activation system including an activation device according to Embodiment 3-2 of the present invention.
FIG. 24 is a flowchart illustrating an operation of the activation system illustrated in FIG. 23.
FIG. 25 is a schematic diagram illustrating a configuration example of an activation system including an activation device according to Embodiment 3-3 of the present invention.
FIG. 26 is a top view illustrating a placement surface of the activation device illustrated in FIG. 25.
FIG. 27 is a schematic diagram illustrating Modified Example of Embodiments 1-1 to 3-3.

### Description of Embodiments

Hereinafter, an activation device according to embodiments of the present invention will be described with reference to the drawings. The present invention is not limited by the embodiments. The same reference signs are used to refer to the same elements throughout the drawings.

### (Embodiment 1-1)

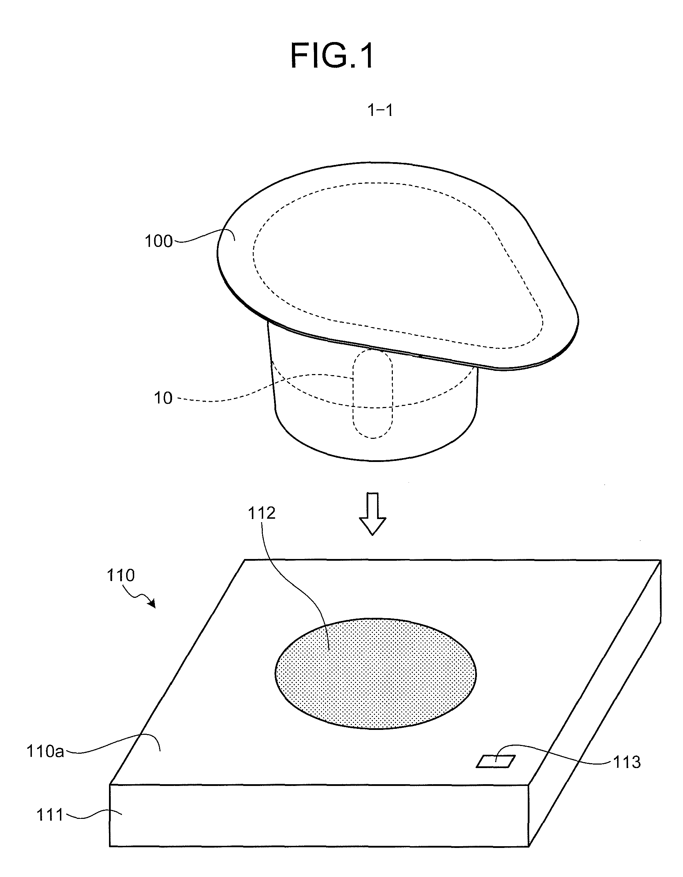

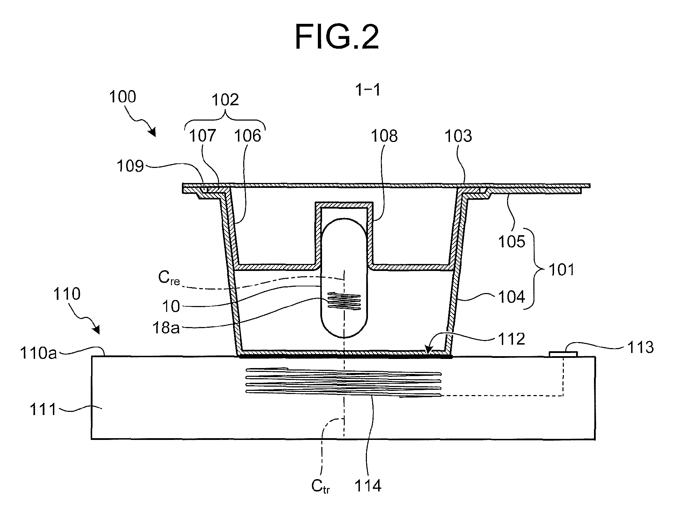

FIG. 1 is a perspective view illustrating an appearance of an activation system including an activation device according to Embodiment 1-1 of the present invention. FIG. 2 is a schematic diagram illustrating a configuration example of the activation system illustrated in FIG. 1. The activation system 1-1 illustrated in FIGs. 1 and 2 includes a capsule endoscope 10 as an example of a capsule medical device, a container 100 that houses the capsule endoscope 10, and an activation device 110 that activates the capsule endoscope 10. In FIG. 2, a receiving coil 18a and a transmitting coil 114, which will be described later, are schematically illustrated and a cross-section is illustrated only for the container 100.

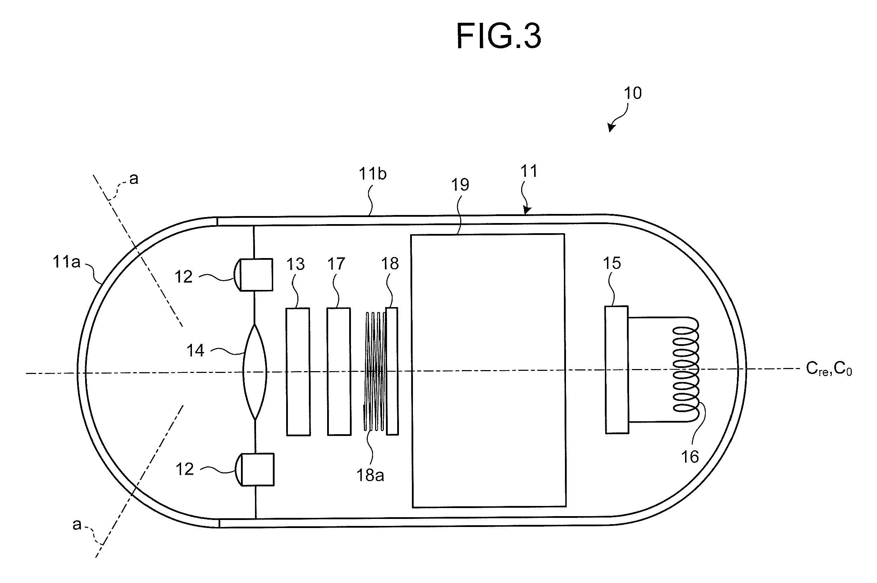

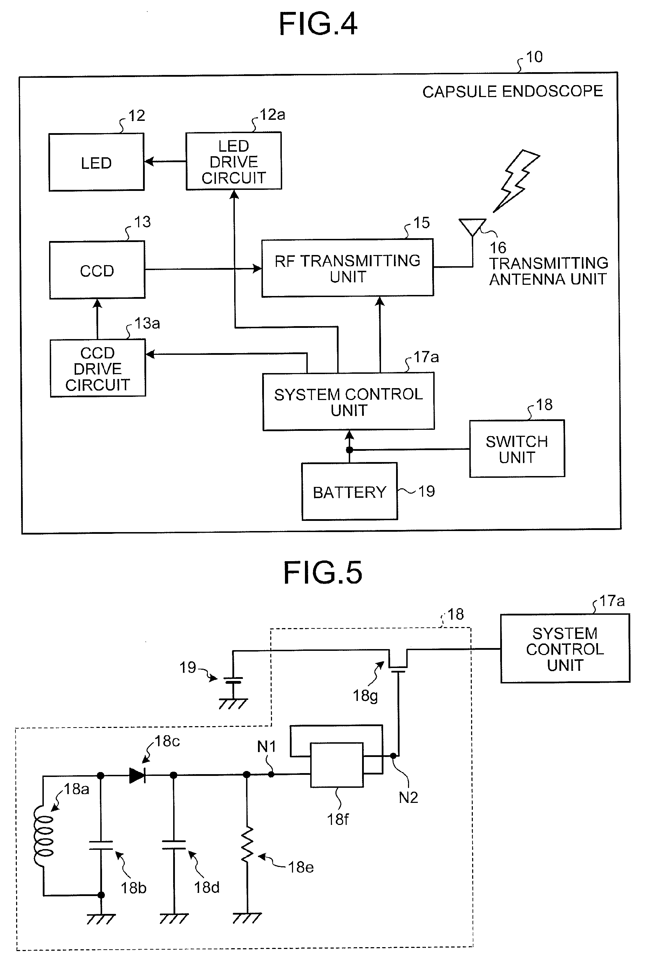

FIG. 3 is a schematic diagram illustrating a schematic configuration of the capsule endoscope 10. FIG. 4 is a block diagram illustrating an internal configuration of the capsule endoscope 10.

As illustrated in FIG. 3, the capsule endoscope 10 includes a closed container 11 which is an outer casing, a plurality of LEDs 12 which are located in the closed container 11 and emit illumination light for illuminating an observed region, a CCD 13 which receives reflected light of the illumination light and captures an image of the observed region, an image forming lens 14 which forms an image of a subject on the CCD 13, an RF transmitting unit 15 which modulates image information acquired by the CCD 13 into an RF signal and transmits the RF signal, a transmitting antenna unit 16 which radiates a radio wave of the RF signal, a control unit 17, a switch unit 18, and a battery 19.

The closed container 11 is an outer casing which has a size that can be swallowed by a human and which liquid-tight seals the inside by elastically fitting together an approximately hemispherical tip cover 11a and a cylindrical body portion cover 11b with a bottom portion. The tip cover 11a has a dome shape and the rear of the dome has a circular opening. The tip cover 11a is formed by a transparent member having transparency or translucency and mirror finishing is applied to a specified range (range indicated by alternate long and short dash lines a and a in FIG. 3) of a surface of the tip cover 11a determined by an image capturing range of the CCD 13. Thereby, the illumination light from the LEDs 12 is enabled to transmit to the outside of the closed container 11 and the reflected light of the illumination light from the subject is enabled to transmit to the inside of the closed container 11.

The body portion cover 11b is a member which includes a cylindrical body portion and an approximately semispherical dome-shaped rear end portion that are integrally formed together and which covers the aforementioned components. The front of the body portion has a circular opening and is fitted to the rear end of the tip cover 11a. The body portion cover 11b is formed by polysulphone or the like that is preferable to secure strength. The body portion cover 11b houses the LEDs 12, the CCD 13, the control unit 17, and the battery 19 in the body portion and houses the RF transmitting unit 15 and the transmitting antenna unit 16 in the rear end portion.

As illustrated in FIG. 4, the capsule endoscope 10 further includes an LED drive circuit 12a that controls a driving state of the LEDs 12, a CCD drive circuit 13a that controls a driving state of the CCD 13, a system control unit 17a that controls operations of the LED drive circuit 12a, the CCD drive circuit 13a, and the RF transmitting unit 15, and a switch unit 18. The LED drive circuit 12a, the CCD drive circuit 13a, and the system control unit 17a are provided in the control unit 17 illustrated in FIG. 3.

The system control unit 17a controls each unit so that the CCD 13 acquires image data of the observed region illuminated by the LEDs 12 while the capsule endoscope 10 is inserted into the subject. The acquired image data is converted into an RF signal by the RF transmitting unit 15 and transmitted to the outside of the subject through the transmitting antenna unit 16. The system control unit 17a has a function to distribute drive power supplied from the battery 19 to other components.

The switch unit 18 includes a receiving coil 18a that generates an induced voltage or an induced current based on a magnetic field applied from the outside. When voltage greater than or equal to a specified value or current greater than or equal to a specified value is generated in the receiving coil 18a, supply of power from the battery 19 to each unit in the capsule endoscope 10 is started and the capsule endoscope 10 is activated. As illustrated in FIG. 3, the receiving coil 18a is provided so that the position and the orientation of the central axis Cᵣₑ of the receiving coil 18a coincide with those of the central axis C₀ of the capsule endoscope 10.

FIG. 5 is a circuit diagram illustrating a configuration example of the switch unit 18. As illustrated in FIG. 5, the switch unit 18 includes a capacitor 18b that forms a resonance circuit with the receiving coil 18a, a diode 18c that forms a rectifier circuit, a smoothing capacitor 18d, a resistor 18e, a frequency dividing circuit 18f, and a power supply switch 18g in addition to the receiving coil 18a. The resonance circuit formed by the receiving coil 18a and the capacitor 18b is adjusted to resonate with a frequency of an AC magnetic field generated by an activation device 110 described later.

When an AC magnetic field is applied to the receiving coil 18a from the outside of the capsule endoscope 10, an alternating current is generated. The alternating current is rectified by the diode 18c, smoothed by the smoothing capacitor 18d, and inputted into the frequency dividing circuit 18f as a direct current electrical signal at a receiving voltage level. The frequency dividing circuit 18f includes a D-type flip-flop circuit (not shown in the drawings) and outputs a signal obtained by dividing the frequency of the inputted electrical signal by 2 to the power supply switch 18g. The power supply switch 18g is formed by a P-channel type FET where the source is connected to the battery 19, the gate is connected to the output of the frequency dividing circuit 18f, and the drain is connected to the system control unit 17a.

When the receiving coil 18a detects an AC magnetic field, a potential at a node N1 becomes a high level. When the potential at the node N1 exceeds a threshold value of the frequency dividing circuit 18f, the output of the frequency dividing circuit 18f (that is, a potential at a node N2) becomes a ground voltage level. Thereby, the power supply switch 18g becomes an on state and the supply of power to each unit in the capsule endoscope 10 is started through the system control unit 17a.

In this way, when the output of the frequency dividing circuit 18f is the ground voltage level, the power supply switch 18g becomes on and the power is supplied from the battery 19 to each unit in the capsule endoscope 10. On the other hand, when the output of the frequency dividing circuit 18f is a power supply voltage level, the power supply switch 18g becomes off and the power is not supplied from the battery 19. That is to say, each time the receiving coil 18a detects an AC magnetic field, the power supply switch 18g performs a toggle operation in which the on/off state is switched. In other words, the frequency dividing circuit 18f functions as a state holding unit of the power supply switch 18g. The frequency dividing circuit 18f is not limited to the D-type flip-flop circuit, but may be, for example, a T-type flip-flop circuit if the frequency dividing circuit 18f can divide the frequency of an input signal by 2.

Next, a structure of the container 100 will be described. As illustrated in FIG. 2, the container 100 is a container that houses the capsule endoscope 10 at a specified position in a specified posture and includes an outer container 101 that can house the capsule endoscope 10 therein, an inner lid portion 102 which is housed in the outer container 101 and which holds the capsule endoscope 10 between itself and the outer container 101, and a sheet-shaped outer lid portion 103 that occludes an opening in the upper surface of the outer container 101. Among them, the outer container 101 and the inner lid portion 102 are manufactured by a forming process such as vacuum forming of polypropylene. Such a container 100 is also called a blister pack.

The outer container 101 includes an approximately cylindrical bottomed housing portion 104 and a handle portion 105 that extends in one direction from an upper end of the housing portion 104. On the other hand, the inner lid portion 102 includes a bottomed cylinder portion 106 whose outer diameter is substantially the same as the inner diameter of the housing portion 104 and an engagement portion 107 that extends toward the outer circumference from the upper end of the cylinder portion 106. At approximately the center of the bottom surface of the cylinder portion 106, a holding portion 108 is provided which is for holding the capsule endoscope 10 and which protrudes toward the opposite of the bottom surface of the housing portion 104. The inner diameter of the holding portion 108 is set to be slightly smaller than the outer diameter of the capsule endoscope 10, so that the holding portion 108 can clamp the capsule endoscope 10. The inner lid portion 102 is held in the outer container 101 in a state in which the inner lid portion 102 floats away from the bottom surface of the housing portion 104 by engaging the engagement portion 107 with a cutout 109 provided near the upper end portion of the outer container 101. Thereby, the position of the inner lid portion 102 with respect to the outer container 101 is determined.

When housing the capsule endoscope 10 in the container 100, the capsule endoscope 10 is inserted into the holding portion 108 from the outer bottom surface of the inner lid portion 102 by adjusting the posture of the capsule endoscope 10 so that the longitudinal direction of the capsule endoscope 10 matches the holding portion 108 and the capsule endoscope 10 is clamped by the holding portion 108. In this state, the inner lid portion 102 is housed in the outer container 101. Thereby, the capsule endoscope 10 is held in a space between the inner lid portion 102 and the outer container 101 in an upright posture. At this time, the receiving coil 18a included in the capsule endoscope 10 is formed so that an opening surface of the receiving coil 18a faces the bottom surface of the container 100 and the central axis Cᵣₑ coincides with the central axis of the bottom surface of the container 100. Further, the opening of the outer container 101 is sealed with the outer lid portion 103 by, for example, a heat sealing process. Thereafter, the container 100 is sterilized by sterilization gas.

In Embodiment 1-1, the container 100 using a method in which the capsule endoscope 10 is clamped by the inner lid portion 102 is described. However, the method of holding the capsule endoscope 10 is not limited if the method can hold the capsule endoscope 10 at a specified position in a specified posture in the container 100. The shape of the housing portion 104 is not limited to an approximately cylindrical shape, but may be, for example, a rectangular parallelepiped shape or a cubic shape.

Next, a configuration of the activation device 110 will be described.

As illustrated in FIGs. 1 and 2, the activation device 110 includes a case 111, a guide display portion 112 provided on the upper surface of the case 111, a switch button 113 provided on an outer side (for example, on the upper surface) of the case 111, and a transmitting coil 114 which is housed in the case 111 and generates a magnetic field when current flows through the transmitting coil 114. The upper surface of the case 111 has a planar shape and the upper surface is a placement surface 110a of the container 100.

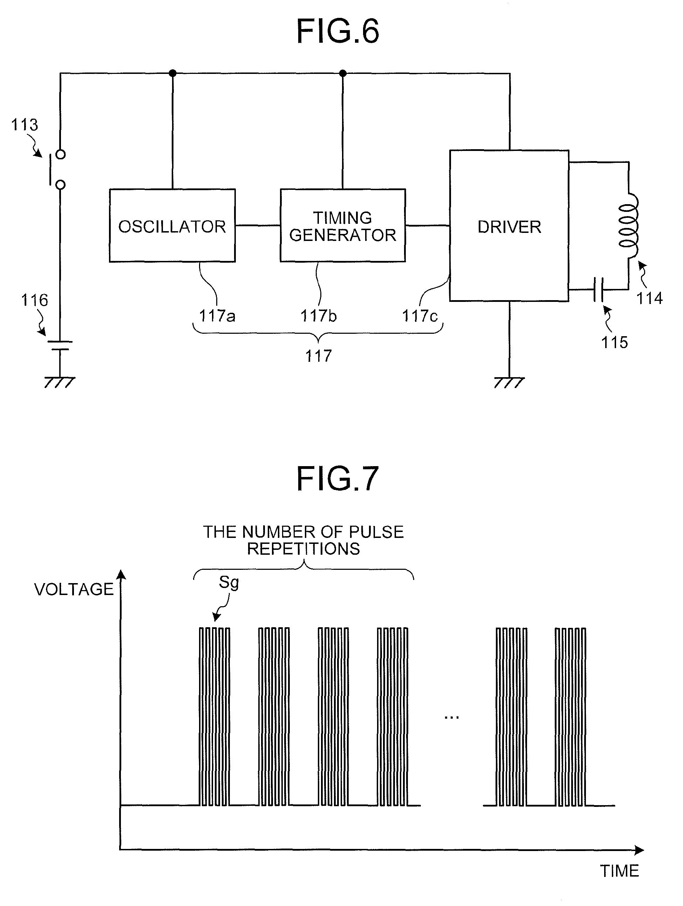

FIG. 6 is a circuit diagram illustrating an internal configuration of the activation device 110. The activation device 110 includes a capacitor 115 that forms a resonance circuit with the transmitting coil 114, a power supply 116 for driving the resonance circuit, and a signal generator 117 including an oscillator 117a, a timing generator 117b, and a driver 117c, in addition to the transmitting coil 114. When the switch button 113 is pressed and power from the power supply 116 is supplied to the signal generator 117, the timing generator 117b converts a signal outputted from the oscillator 117a into a signal of a specified frequency and inputs the signal into the driver 117c. The driver 117c drives the resonance circuit formed by the transmitting coil 114 and the capacitor 115 based on the inputted signal. Thereby, the transmitting coil 114 generates an AC magnetic field of a specified frequency.

As illustrated in FIG. 2, the transmitting coil 114 is arranged at a position a specified distance away from the placement surface 110a with its opening facing the placement surface 110a. In other words, the central axis Cₜᵣ of the transmitting coil 114 is perpendicular to the placement surface 110a. To reduce the power consumption of the activation device 110 when activating the capsule endoscope 10, the transmitting coil 114 should be close to the placement surface 110a as much as possible.

The guide display portion 112 is a guide portion that indicates a specific position on the placement surface 110a to which the container 100 should be placed. Hereinafter, in the present specification, the specific position is a position where the receiving coil 18a included in the capsule endoscope 10 can generate an induced current or an induced voltage greater than or equal to a specified value (current sufficient to turn on the power supply switch 18g) when the container 100 housing the capsule endoscope 10 is placed on the placement surface 110a and a magnetic field is generated by applying current to the transmitting coil 114. At this time, the transmitting coil 114 and the receiving coil 18a are in a state in which their central axes Cₜᵣ and Cᵣₑ are substantially coincident with each other and their openings face each other with a specified distance in between. In Embodiment 1-1, the guide display portion 112 is formed by changing color of a circular area, whose size is substantially the same as that of the bottom of the outer container 101 and whose center is the central axis Cₜᵣ of the transmitting coil 114, from that of surrounding areas.

Next, an activation method of the capsule endoscope 10 will be described.

First, a user places the container 100 that houses the capsule endoscope 10 on the guide display portion 112 provided on the placement surface 110a of the activation device 110. Thereby, the transmitting coil 114 and the receiving coil 18a are brought into a state in which their central axes Cᵣₑ and Cₜᵣ are substantially coincident with each other and the openings of the coils face each other with a specified distance in between. The positional relationship (distance) between the transmitting coil 114 and the receiving coil 18a in the vertical direction at this time is determined in advance by defining the distance between the transmitting coil 114 and the placement surface 110a and the position of holding the capsule endoscope 10 in the container 100.

In this condition, when the user presses the switch button 113, the activation device 110 generates an AC magnetic field from the transmitting coil 114. As a result, current greater than or equal to a specified value is generated in the receiving coil 18a by electromagnetic induction and the switch unit 18 is brought into an on state.

The switch unit 18 may be brought into an on state when the activation device 110 intermittently outputs an AC magnetic field from the transmitting coil 114 in a predefined pattern and the system control unit 17a of the capsule endoscope 10 determines that a signal pattern of a detected magnetic field (that is, a signal pattern of voltage or current generated by the receiving coil 18a due to application of the magnetic field) is coincident with the predefined pattern. For example, when a signal Sg having a pattern as illustrated in FIG. 7 is detected and the system control unit 17a determines that the number of pulse repetitions exceeds a specified number, the system control unit 17a brings the switch unit 18 into an on state.

As described above, according to Embodiment 1-1, it is possible to activate the capsule endoscope 10 housed in the container 100 in a state in which the container 100 is placed on the placement surface 110a of the activation device 110. Therefore, it is possible to reduce the size of the activation device as compared with a method in which the capsule endoscope 10 is activated by causing the capsule endoscope 10 to pass through the transmitting coil, so that it is possible to reduce the power consumption.

Further, according to Embodiment 1-1, the guide display portion 112 is provided on the placement surface 110a, so that the position where the container 100 is to be placed is obvious. Therefore, it is possible to suppress a position shift between the transmitting coil 114 in the activation device 110 and the receiving coil 18a in the capsule endoscope 10. Thus, it is possible to easily and reliably activate the capsule endoscope 10 by only pressing the switch button 113.

Further, according to Embodiment 1-1, it is not necessary to perform an operation to search for the position where the container 100 is to be placed in a state in which power is supplied to the signal generator 117, so that it is possible to suppress useless power consumption and efficiently activate the capsule endoscope 10.

In Embodiment 1-1, the color of the guide display portion 112 is changed from that of the surrounding areas. However, the guide display portion 112 may have any form as long as a user can recognize the position where the container 100 is to be placed. For example, the area where the container 100 is to be placed may be simply surrounded by a line or a material having a texture different from that of other areas may be attached to the area.

### (Embodiment 1-2)

Next, Embodiment 1-2 of the present invention will be described.

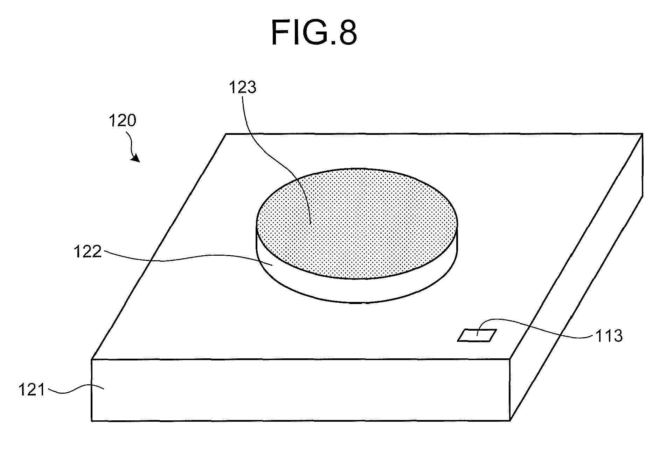

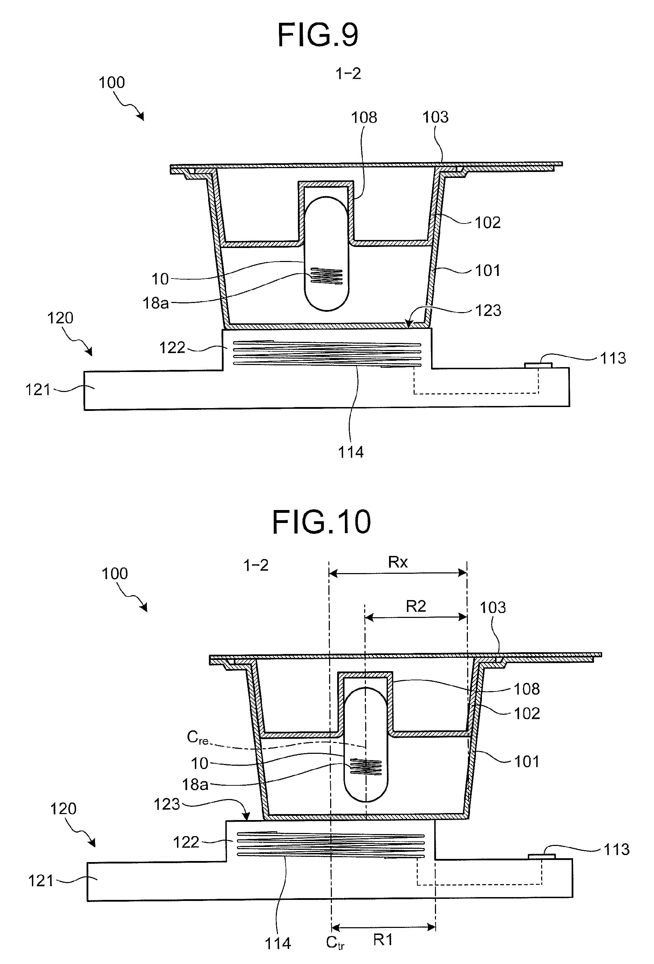

FIG. 8 is a perspective view illustrating an appearance of an activation device according to Embodiment 1-2. FIG. 9 is a schematic diagram illustrating a configuration example of an activation system including the activation device illustrated in FIG. 8. The activation system 1-2 illustrated in FIG. 9 includes a capsule endoscope 10, a container 100 that houses the capsule endoscope 10, and an activation device 120 that activates the capsule endoscope 10. In FIG. 9, a receiving coil 18a and a transmitting coil 114 are schematically illustrated and a cross-section is illustrated only for the container 100.

The activation device 120 includes a case 121 where a convex portion 122 is provided on a part of its upper surface, a switch button 113 provided on an outer side of the case 121, and a transmitting coil 114 which is housed in the case 121 and generates a magnetic field when current flows through the transmitting coil 114. An internal configuration of the activation device 120 including the transmitting coil 114 is the same as that of Embodiment 1-1 (see FIG. 6).

The upper surface of the convex portion 122 has a planar shape. The upper surface is a placement portion 123 on which the container 100 is to be placed. The position of the convex portion 122 is set to a position where an induced current or an induced voltage greater than or equal to a specified value can be generated in the receiving coil 18a in the capsule endoscope 10 based on the magnetic field generated by the transmitting coil 114 when the container 100 is placed on the placement portion 123. Specifically, a circular area, whose size is substantially the same as that of the bottom of the outer container 101 and whose center is the central axis Cᵣₑ of the transmitting coil 114, is defined as the cylindrical convex portion 122. In other words, the convex portion 122 functions as a guide portion that guides the container 100 to a specific position by its outer circumference when activating the capsule endoscope 10.

Next, a condition of the convex portion 122 given in order to more reliably activate the capsule endoscope 10 will be described with reference to FIG. 10. As illustrated in FIG. 10, when the radius of the convex portion 122 is R1 and the radius of the bottom of the container 100 is R2, the diameter of the convex portion 122 is defined so that R1 ≤ R2 is established. When a range of the central axis Cᵣₑ of the receiving coil 18a where the capsule endoscope 10 can be activated even when the transmitting coil 114 and the receiving coil 18a are shifted from each other in the horizontal direction is smaller than or equal to a radius Rx, the radius Rx is set so that R1 < Rx is established. Thereby, it is possible to activate the capsule endoscope 10 as long as the container 100 is in a posture to be able to stand upright on the placement portion 123 (that is, as long as the central axis Cᵣₑ does not go beyond the circumference of the convex portion 122). The activation method of the capsule endoscope 10 is the same as that in Embodiment 1-1.

As described above, according to Embodiment 1-2, the convex portion 122 whose upper surface is the placement portion 123 of the container 100 is provided to the activation device 120, so that it is possible to easily grasp a position shift between the transmitting coil 114 in the activation device 110 and the receiving coil 18a in the capsule endoscope 10 and suppress the position shift. Therefore, it is possible to easily and reliably activate the capsule endoscope 10 by only pressing the switch button 113.

### (Embodiment 1-3)

Next, Embodiment 1-3 of the present invention will be described.

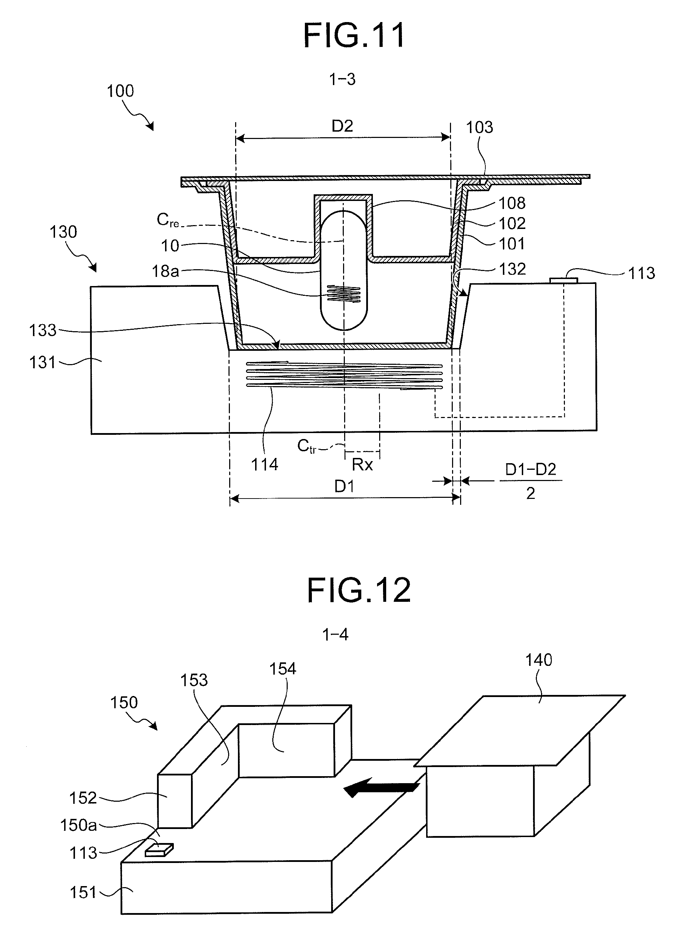

FIG. 11 is a cross-sectional view illustrating a configuration example of an activation system including an activation device according to Embodiment 1-3. The activation system 1-3 illustrated in FIG. 11 includes a capsule endoscope 10, a container 100 that houses the capsule endoscope 10, and an activation device 130 that activates the capsule endoscope 10. In FIG. 11, a receiving coil 18a and a transmitting coil 114 are schematically illustrated and hatching that indicates a cross-section of a case 131 described later is omitted.

The activation device 130 includes a case 131 where a concave portion 132 is provided in a part of its upper surface, a switch button 113 provided on an outer side of the case 131, and a transmitting coil 114 which is housed in the case 131 and generates a magnetic field when current flows through the transmitting coil 114. An internal configuration of the activation device 130 including the transmitting coil 114 is the same as that of Embodiment 1-1 (see FIG. 6).

The bottom surface of the concave portion 132 has a planar shape. The bottom surface is a placement portion 133 on which the container 100 is to be placed. The position of the concave portion 132 is set to a position where an induced current or an induced voltage greater than or equal to a specified value can be generated in the receiving coil 18a in the capsule endoscope 10 based on the magnetic field generated by the transmitting coil 114 when the container 100 is placed on the concave portion 132. Specifically, an area, whose bottom has a circular shape whose size is substantially the same as that of the bottom of the outer container 101 and whose center is the central axis Cᵣₑ of the transmitting coil 114, is defined as the concave portion 132. In other words, the concave portion 132 functions as a guide portion that guides the container 100 to a specific position when the capsule endoscope 10 is activated.

Next, a condition of the concave portion 132 given in order to more reliably activate the capsule endoscope 10 will be described. As illustrated in FIG. 11, the diameter of the bottom of the concave portion 132 is D1 and the diameter of the bottom of the container 100 is D2 (D2 ≤ D1). When a range of the central axis Cᵣₑ of the receiving coil 18a where the capsule endoscope 10 can be activated even when the transmitting coil 114 and the receiving coil 18a are shifted from each other in the horizontal direction is smaller than or equal to a radius Rx, the radius Rx is set so that (D1 - D2)/2 < Rx is established. Thereby, when the container 100 is inserted horizontally to the bottom of the concave portion 132 (placement portion 133), it is possible to reliably activate the capsule endoscope 10. The activation method of the capsule endoscope 10 is the same as that in Embodiment 1-1.

As described above, according to Embodiment 1-3, the concave portion 132 whose bottom surface is the placement portion 133 of the container 100 is provided to the activation device 130, so that it is possible to easily suppress a position shift between the transmitting coil 114 in the activation device 130 and the receiving coil 18a in the capsule endoscope 10 by a simple operation to put the container 100 in the concave portion 132. Therefore, it is possible to easily and reliably activate the capsule endoscope 10 by only pressing the switch button 113.

### (Embodiment 1-4)

Next, Embodiment 1-4 of the present invention will be described.

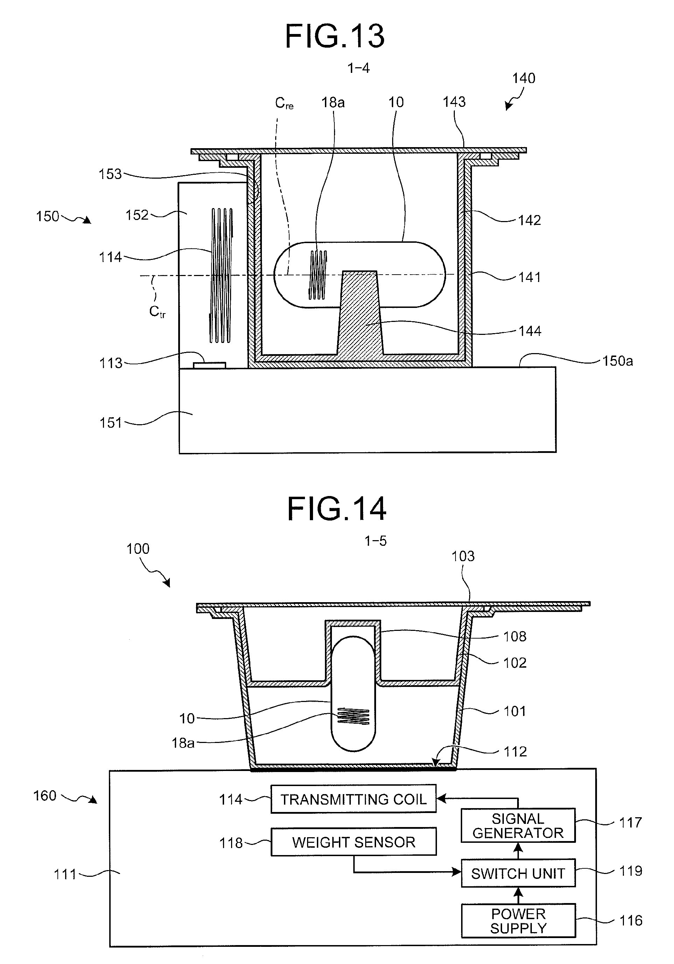

FIG. 12 is a perspective view illustrating an appearance of an activation system including an activation device according to Embodiment 1-4. FIG. 13 is a schematic diagram illustrating a configuration example of the activation system illustrated in FIG. 12. The activation system 1-4 illustrated in FIGs. 12 and 13 includes a capsule endoscope 10, a container 140 that houses the capsule endoscope 10, and an activation device 150 that activates the capsule endoscope 10. In FIG. 13, a receiving coil 18a and a transmitting coil 114 are schematically illustrated and a cross-section is illustrated only for the container 140.

The container 140 includes a container main body 141 having an approximately rectangular parallelepiped shape, an inner container 142 housed in the container main body 141, and a sheet-shaped outer lid portion 143 that occludes an opening in the upper surface of the container main body 141. In the inner container 142, a holding portion 144 that holds the capsule endoscope 10 at a specified position in a specified posture is formed. In Embodiment 1-4, the holding portion 144 clamps the body portion of the capsule endoscope 10 so that the central axis Cᵣₑ of the receiving coil 18a is in parallel with the bottom surface of the container 140.

The activation device 150 includes a pedestal 151 whose upper surface is a placement surface 150a of the container 140 and a wall portion 152 provided on the pedestal 151 and integrally with the pedestal 151. The pedestal 151 and the wall portion 152 are the case of the activation device 150.

The activation device 150 includes a switch button 113 provided on the pedestal 151 and a transmitting coil 114 which generates a magnetic field when current flows through the transmitting coil 114. The transmitting coil 114 is provided in the wall portion 152 such that the central axis Cₜᵣ is in parallel with the placement surface 150a. An internal configuration of the activation device 150 is the same as that of Embodiment 1 (see FIG. 6).

Two side surfaces of the wall portion 152 are abutting surfaces 153 and 154, against which adjacent side surfaces of the container 140 are abutted. The abutting surfaces 153 and 154 are provided at a position where, when the container 140 is abutted against the abutting surfaces 153 and 154, the transmitting coil 114 and the receiving coil 18a are brought into a state in which their central axes Cᵣₑ and Cₜᵣ are substantially coincident with each other and the openings of the coils face each other with a specified distance in between. Thereby, it is possible to cause the receiving coil 18a to generate an induced current or an induced voltage greater than or equal to a specified value based on a magnetic field generated by the transmitting coil 114. As a result, when the capsule endoscope 10 housed in the container 140 is activated, the abutting surfaces 153 and 154 function as a guide portion that guides the container 140 to a specific position.

As described above, according to Embodiment 1-4, the abutting surfaces 153 and 154, against which the container 140 is abutted, are provided to the activation device 150, so that it is possible to easily suppress a position shift between the transmitting coil 114 in the activation device 150 and the receiving coil 18a in the capsule endoscope 10 by a simple operation to cause the container 140 to abut against the abutting surfaces 153 and 154. Therefore, it is possible to easily and reliably activate the capsule endoscope 10 by only pressing the switch button 113.

### (Modified Example 1-1)

Next, Modified Example 1-1 of Embodiments 1-1 to 1-4 will be described.

FIG. 14 is a schematic diagram illustrating a configuration example of an activation system including an activation device according to Modified Example 1-1. The activation system 1-5 illustrated in FIG. 14 includes a capsule endoscope 10, a container 100 that houses the capsule endoscope 10, and an activation device 160 that activates the capsule endoscope 10. In FIG. 14, a receiving coil 18a is schematically illustrated and a cross-section is illustrated only for the container 100. The internal configuration of the activation device 160 is illustrated by a block diagram.

In the same manner as the activation device 110 according to Embodiment 1-1, the activation device 160 includes a case 111 in which a guide display portion 112 is provided on its upper surface which is the placement surface of the container 100. The activation device 160 includes a weight sensor 118 and a switch unit 119 instead of the switch button 113 in the circuit configuration included in the activation device 110 (see FIG. 6). In FIG. 14, the capacitor 115 (see FIG. 6) that forms a resonance circuit with the transmitting coil 114 is omitted.

When the container 100 is placed on the activation device 160, the weight sensor 118 detects the weight of the container 100 and outputs a detection signal. Accordingly, the switch unit 119 is turned on and power supply from the power supply 116 to the signal generator 117 is started. Thereby, the transmitting coil 114 generates an AC magnetic field and the capsule endoscope 10 housed in the container 100 is activated.

As described above, according to Modified Example 1-1, when the container 100 is placed on the activation device 160, the activation device 160 starts operation, so that it is possible to quickly activate the capsule endoscope 10 by a simple operation.

In Modified Example 1-1, an example is described in which the weight sensor 118 and the switch unit 119 are provided to the activation device 110 according to Embodiment 1-1. However, the same configuration may be applied to the activation devices 120, 130, and 150 according to Embodiments 1-2 to 1-4. A pressure sensor may be employed instead of the weight sensor 118.

### (Modified Example 1-2)

In Embodiments 1-1 to 1-4 described above, a magnetic field applied to the receiving coil 18a included in the capsule endoscope 10 is generated by applying current to the transmitting coil 114. Instead, a permanent magnet may be provided in the activation device. Also in this case, it is possible to easily and reliably activate the capsule endoscope 10 by providing the guide display portion 112 (see FIG. 1), the convex portion 122 (see FIG. 8), the concave portion 132 (see FIG. 11), the wall portion 152 (see FIG. 12), or the like on the placement surface on which the container 100 is placed.

### (Embodiment 2-1)

Next, Embodiment 2-1 of the present invention will be described.

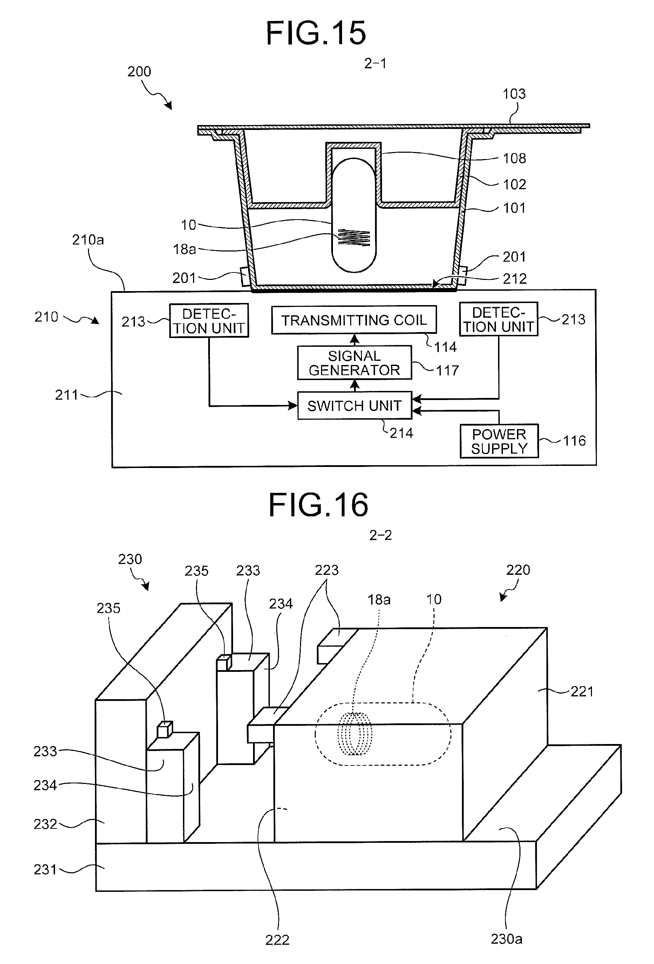

FIG. 15 is a schematic diagram illustrating a configuration example of an activation system including an activation device according to Embodiment 2-1. The activation system 2-1 illustrated in FIG. 15 includes a capsule endoscope 10, a container 200 that houses the capsule endoscope 10, and an activation device 210 that activates the capsule endoscope 10. The container 200 has a configuration in which portions to be detected 201 are provided near the bottom of the outer container 101 of the container 100 described in Embodiment 1-1. In FIG. 15, a receiving coil 18a is schematically illustrated and a cross-section is illustrated only for the container 200. The internal configuration of the activation device 210 is illustrated by a block diagram.

The portion to be detected 201 is a member that can be detected by a detection unit 213 included in the activation device 210 described later. Specifically, the portion to be detected 201 may be a magnet that can be detected by a magnetic sensor, a conductor that can be detected by a conduction check circuit, a metal that can be detected by a metal detection sensor, a reflecting plate that reflects infrared light and the like, and a mechanical member that can be detected by a weight sensor, a pressure sensor, a pressing force sensor, or the like. It is preferable that the portion to be detected 201 is provided at a plurality of positions around the bottom of the outer container 101. In Embodiment 2-1, the portion to be detected 201 is provided at two positions opposite to each other on the circumference of the outer container 101.

On the other hand, the activation device 210 includes a case 211 whose upper surface is a placement surface 210a of the container 200. A guide display portion 212 that indicates a specific position on which the container 200 should be placed is provided on the placement surface 210a. The appearance of the guide display portion 212 is the same as that of the guide display portion 112 illustrated in FIG. 1.

The activation device 210 includes a detection unit 213 and a switch unit 214 instead of the switch button 113 in the circuit configuration (see FIG. 6) included in the activation device 110 according to Embodiment 1-1. In FIG. 15, the capacitor 115 (see FIG. 6) that forms a resonance circuit with the transmitting coil 114 is omitted.

The detection unit 213 is a sensor that detects the portion to be detected 201 provided to the container 200 and outputs a detection signal, and the detection unit 213 is also a recognition unit that recognizes the position of the container 200 (that is, the capsule endoscope 10) through the portion to be detected 201. The detection unit 213 is formed by a magnetic sensor that can detect a magnet, a conduction check circuit that can detect a conductor, a metal detection sensor that can detect a metal, an optical sensor that can detect infrared light, or a weight sensor, a pressure sensor, a pressing force sensor, or the like that can detect a mechanical member according to the configuration of the portion to be detected 201. The detection unit 213 is provided at a position where the portion to be detected 201 can be detected when the container 200 is placed at a defined position on the placement surface 210a (that is, on the guide display portion 212). In Embodiment 2-1, the detection unit 213 is provided at a plurality of positions (for example, four positions) near the circumference of the guide display portion 212.

The switch unit 214 is turned on when the switch unit 214 receives the detection signal outputted from the detection unit 213. Thereby, power supply from the power supply 116 to the signal generator 117 and the transmitting coil 114 is started.

Next, the activation method of the capsule endoscope 10 will be described.

First, a user places the container 200 that houses the capsule endoscope 10 on the placement surface 210a of the activation device 210 by using the guide display portion 212 as a target.

When the detection unit 213 detects the portion to be detected 201, the switch unit 214 is turned on, and power supply from the power supply 116 to the signal generator 117 and the transmitting coil 114 is started. Thereby, an AC magnetic field is generated from the transmitting coil 114 and the capsule endoscope 10 housed in the container 200 is activated. The AC magnetic field generated by the transmitting coil 114 may be a magnetic field having strength greater than or equal to a specified value or may be a magnetic field having a specified pattern. When the capsule endoscope 10 detects a magnetic field of strength greater than or equal to a specified value or detects a magnetic field of a specified pattern, the capsule endoscope 10 becomes ON state.

Thereafter, when the detection unit 213 does not detect the portion to be detected 201 anymore, the switch unit 214 is turned off.

On the other hand, when the container 200 is not placed on the specific position even though the container 200 is placed on the placement surface 210a, the detection unit 213 does not detect the portion to be detected 201, so that the capsule endoscope 10 is not activated. In this case, the user may check the guide display portion 212 and adjust the position of the container 200.

As described above, according to Embodiment 2-1, only when the container 200 is placed on the specific position on the placement surface 210a and the capsule endoscope 10 is arranged at a position where the capsule endoscope 10 can be activated, the detection unit 213 detects the portion to be detected 201 and starts power supply to the signal generator 117. Therefore, it is possible to suppress consumption of the power supply 116 of the activation device 210.

### (Embodiment 2-2)

Next, Embodiment 2-2 of the present invention will be described.

FIG. 16 is a perspective view illustrating a configuration example of an activation system including an activation device according to Embodiment 2-2. The activation system 2-2 illustrated in FIG. 16 includes a capsule endoscope 10, a container 220 that houses the capsule endoscope 10, and an activation device 230 that activates the capsule endoscope 10.

The container 220 includes a container main body 221 having an approximately rectangular parallelepiped shape. In the container main body 221, a holding portion (not shown in the drawings) that holds the capsule endoscope 10 at a specified position in a specified posture is provided. On one outer side surface 222 of the container main body 221, portions to be detected 223 which protrude from the side surface 222 are provided. The portion to be detected 223 is a member that can be detected by a detection unit 235 described later. In Embodiment 2-2, the portion to be detected 223 is formed by a convex portion that is mechanically detected by a pressing force sensor. In Embodiment 2-2, two portions to be detected 223 are provided. However, the number of the portions to be detected 223 is not limited to two, but may be one or three or more.

On the other hand, the activation device 230 includes a pedestal 231 and a wall portion 232 provided on the pedestal 231 and integrally with the pedestal 231. The upper surface of the pedestal 231 has a planar shape and the upper surface is a placement surface 230a of the container 220. The pedestal 231 and the wall portion 232 form a case of the activation device 230 and the activation device 230 includes a transmitting coil (not shown in the drawings) which generates a magnetic field when current flows through the transmitting coil.

The wall portion 232 is provided with two abutting members 233 including an abutting surface 234 against which the side surface 222 of the container main body 221 is abutted. The abutting surfaces 234 are provided at positions where, when the container 220 is abutted against the abutting surfaces 234, the axis of the transmitting coil (not shown in the drawings) included in the activation device 230 and the axis of the receiving coil 18a are substantially coincident with each other and the openings of the coils face each other with a specified distance in between. Thereby, it is possible to cause the receiving coil 18a to generate an induced current or an induced voltage greater than or equal to a specified value based on a magnetic field generated by the transmitting coil 114.

Each abutting member 233 is provided with the detection unit 235 that can detect the portion to be detected 223. Specifically, the detection unit 235 is a pressure sensor. The internal configuration of the activation device 230 other than the detection unit 235 is the same as that in Embodiment 2-1.

Next, the activation method of the capsule endoscope 10 will be described.

First, a user places the container 220 that houses the capsule endoscope 10 on the pedestal 231, slides the container 220 toward the wall portion 232, and causes the side surface 222 of the container 220 to abut against the abutting surfaces 234. When the detection unit 235 detects the portion to be detected 223, the activation device 230 starts power supply to the transmitting coil 114 to generate an AC magnetic field. Thereby, the capsule endoscope 10 is activated. The AC magnetic field generated by the activation device 230 may be a magnetic field of strength greater than or equal to a specified value or may be a magnetic field having a specified pattern. When the capsule endoscope 10 detects a magnetic field of strength greater than or equal to a specified value or detects a magnetic field of a specified pattern, the capsule endoscope 10 becomes ON state.

Thereafter, when the detection unit 235 does not detect the portion to be detected 223 anymore, the activation device 230 stops the power supply to the transmitting coil 114.

As described above, according to Embodiment 2-2, it is possible to achieve an appropriate positional relationship between the receiving coil 18a in the capsule endoscope 10 and the transmitting coil in the activation device 230 by causing the container 220 to abut against the abutting surfaces 234 of the activation device 230. Therefore, the user can easily and reliably activate the capsule endoscope 10. The power supply to the transmitting coil is started only when the positional relationship between the receiving coil 18a and the transmitting coil is appropriate, so that it is possible to suppress consumption of the power supply of the activation device 230.

### (Embodiment 2-3)

Next, Embodiment 2-3 of the present invention will be described.

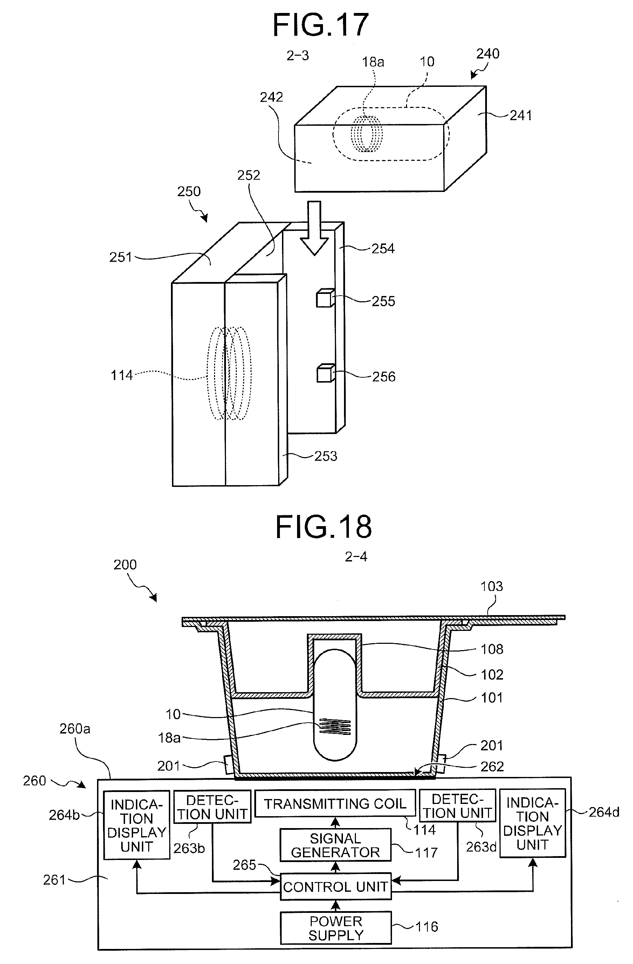

FIG. 17 is a perspective view illustrating a configuration example of an activation system including an activation device according to Embodiment 2-3. The activation system 2-3 illustrated in FIG. 17 includes a capsule endoscope 10, a container 240 that houses the capsule endoscope 10, and an activation device 250 that activates the capsule endoscope 10.

The container 240 includes a container main body 241 having an approximately rectangular parallelepiped shape. In the container main body 241, a holding portion (not shown in the drawings) that holds the capsule endoscope 10 at a specified position in a specified posture is provided.

On the other hand, the activation device 250 includes a case 251 including a transmitting coil 114 which generates a magnetic field when current flows through the transmitting coil 114 and guide walls 253 and 254 provided integrally with the case 251.. Among them, one guide wall 254 is provided with detection units 255 and 256 which detect the container 240. The internal configuration of the activation device 250 other than the detection units 255 and 256 is the same as that in Embodiment 2-1. In Embodiment 2-3, configurations of the detection units 255 and 256 are not limited particularly. For example, an optical sensor that detects infrared light reflected by the container 240 can be applied as the detection units 255 and 256.

The guide walls 253 and 254 are guide portions that prevent lateral displacement from a slide direction when sliding the container 240 in a direction indicated by an arrow in FIG. 17 while causing a specified side surface 242 of the container 240 to abut against a side surface 252 of the case 251.

The detection unit 255 is provided at a position where, when the container 240 is slid in the direction indicated by the arrow in FIG. 17, an induced current or an induced voltage greater than or equal to a specified value can be generated in the receiving coil 18a included in the capsule endoscope 10 based on the magnetic field generated by the transmitting coil 114 provided in the case 251. On the other hand, the detection unit 256 is provided at a position where, when the container 240 is further slid after an induced current or an induced voltage greater than or equal to the specified value is once generated in the receiving coil 18a, an induced current or an induced voltage greater than or equal to the specified value cannot be generated in the receiving coil 18a based on the magnetic field generated by the transmitting coil 114.

Next, the activation method of the capsule endoscope 10 will be described.

First, a user causes the specified side surface 242 of the container 240 that houses the capsule endoscope 10 to abut the side surface 252 of the case 251 and slides the container 240 in a direction indicated by an arrow in FIG. 17. When the detection unit 255 detects the container 240 during the above operation, the activation device 250 generates an AC magnetic field by starting power supply to the transmitting coil 114. Thereby, the capsule endoscope 10 is activated. The AC magnetic field generated by the activation device 250 may be a magnetic field of strength greater than or equal to a specified value or may be a magnetic field having a specified pattern. When the capsule endoscope 10 detects a magnetic field of strength greater than or equal to a specified value or detects a magnetic field of a specified pattern, the capsule endoscope 10 becomes ON state.

Thereafter, when the detection unit 256 detects the container 240, the activation device 250 stops the power supply to the transmitting coil 114.

As described above, according to Embodiment 2-3, an appropriate positional relationship between the receiving coil 18a in the capsule endoscope 10 and the transmitting coil 114 in the activation device 250 is achieved by causing the container 240 to abut against the side surface 252 and sliding the container 240 between the guide walls 253 and 254, so that the user can easily and reliably activate the capsule endoscope 10. Further, when an appropriate positional relationship between the receiving coil 18a and the transmitting coil 114 is achieved, the power supply to the transmitting coil 114 is started and an AC magnetic field is generated, so that it is possible to suppress consumption of the power supply of the activation device 250.

In FIG. 17, two detection units 255 and 256 are provided. However, at least one detection unit may be provided. When there is one detection unit, the activation device 250 may supply power to the transmitting coil 114 while the detection unit detects the container 240, and the activation device 250 may stop the power supply to the transmitting coil 114 when the detection unit does not detect the container 240 anymore.

In Embodiment 2-3, the container 240 is slid in a vertical direction. However, the container 240 may be slid in a horizontal direction. In Embodiment 2-3, the container 240 is slid in a plane in parallel with the opening surfaces of the transmitting coil 114 and the receiving coil 18a. However, the container 240 may be slid in a direction in parallel with the axes of these coils.

### (Embodiment 2-4)

Next, Embodiment 2-4 of the present invention will be described.

FIG. 18 is a schematic diagram illustrating a configuration example of an activation system including an activation device according to Embodiment 2-4. The activation system 2-4 illustrated in FIG. 18 includes a capsule endoscope 10, a container 200 that houses the capsule endoscope 10, and an activation device 260 that activates the capsule endoscope 10. In FIG. 18, a receiving coil 18a is schematically illustrated and a cross-section is illustrated only for the container 200. The internal configuration of the activation device 260 is illustrated by a block diagram.

The activation device 260 includes a case 261. The upper surface of the case 261 has a planar shape and the upper surface is a placement surface 260a of the container 200. A guide display portion 262 that indicates a specific position on which the container 200 should be placed may be provided on the placement surface 260a.

The activation device 260 includes a plurality of detection units 263a to 263d, a plurality of indication display units 264a to 264d, and a control unit 265 instead of the switch button 113 in the circuit configuration (see FIG. 6) included in the activation device 110 according to Embodiment 1-1. The configurations and the operations of the transmitting coil 114 to the signal generator 117 are the same as those in the in Embodiment 1. In FIG. 18, the capacitor 115 (see FIG. 6) that forms a resonance circuit with the transmitting coil 114 is omitted.

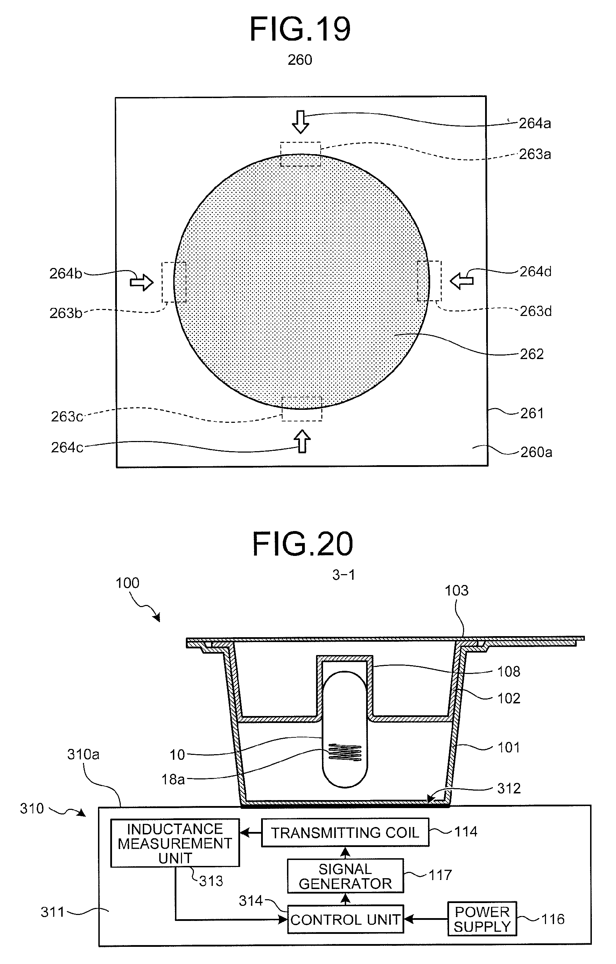

FIG. 19 is a top view illustrating the placement surface 260a of the activation device 260. As illustrated in FIG. 19, a plurality of indication display units 264a to 264d are arranged around the guide display portion 262 on the placement surface 260a. A plurality of detection units 263a to 263d are provided in the case 261.

The detection units 263a to 263d are sensors that detect the portion to be detected 201 provided to the container 200 and output a detection signal, and the detection units 263a to 263d are also recognition units that recognize the position of the container 200 (that is, the capsule endoscope 10) through the portion to be detected 201. Each of the detection units 263a to 263d is formed by a magnetic sensor, a conduction check circuit, a metal detection sensor, an optical sensor, a weight sensor, a pressure sensor, a pressing force sensor, or the like according to the configuration of the portion to be detected 201. These detection units 263a to 263d are provided at positions where the portion to be detected 201 can be detected when the container 200 is placed on a specified position on the placement surface 260a. In Embodiment 2-4, the detection units 263a to 263d are provided at four positions near the circumference of the guide display portion 262.

The indication display units 264a to 264d are notification units that notify a user that the placement position of the container 200 is not appropriate when the container 200 on the placement surface 260a is shifted from the specific position, and the indication display units 264a to 264d are also guidance units for guiding the container 200 to the specific position. In Embodiment 2-4, four arrow-shaped areas indicating a direction of the guide display portion 262 are used as the indication display units 264a to 264d. These indication display units 264a to 264d include, for example, a light-emitting element such as an LED and light up under control of the control unit 265. The number of the indication display units 264a to 264d is not limited to four, and the shape of the areas that light up is not limited to an arrow shape. Each of the indication display units 264a to 264d may be configured to be able to light up with a plurality of colors (for example, red and green).

The control unit 265 controls a lighting-up operation of the indication display units 264a to 264d and an operation of the signal generator 117 based on a detection result that the detection units 263a to 263d detect the portion to be detected 201.

Next, the activation method of the capsule endoscope 10 will be described.

First, a user places the container 200 that houses the capsule endoscope 10 on the placement surface 260a of the activation device 260 by using the guide display portion 262 as a target.

The control unit 265 detects a shift of the container 200 from the specific position from the detection results of the portions to be detected 201 by the detection units 263a to 263d. For example, among the four detection units 263a to 263d, when only the detection unit 263d located on the right side in FIG. 19 detects the portion to be detected 201, it is assumed that the container 200 is shifted right from an appropriate placement position.

In this case, the control unit 265 lights up the indication display units 264a to 264d that indicate a direction in which the container 200 should be moved. For example, specifically, when the container 200 is shifted right from the specific position, the control unit 265 lights up the indication display unit 264d that indicates a left direction. In this case, the control unit 265 may light up the indication display unit 264d with a red color or blink the indication display unit 264d in order to call user's attention. Thereby, the user can recognize that the user should move the container 200 in a left direction.

When all the detection units 263a to 263d detect the portions to be detected 201, the control unit 265 determines that the container 200 is located at the specific position and starts power supply from the power supply 116 to the signal generator 117. Thereby, the transmitting coil 114 generates an AC magnetic field and the capsule endoscope 10 housed in the container 200 is activated. In this case, the control unit 265 may notify the user that the container 200 is appropriately placed and the capsule endoscope 10 is able to be activated by, for example, causing all the indication display units 264a to 264d to emit green light.

The AC magnetic field generated by the transmitting coil 114 may be a magnetic field having strength greater than or equal to a specified value or may be a magnetic field having a specified pattern. When the capsule endoscope 10 detects a magnetic field of strength greater than or equal to a specified value or detects a magnetic field of a specified pattern, the capsule endoscope 10 becomes ON state.

As described above, according to Embodiment 2-4, even when the container 200 on the placement surface 260a is shifted from the specific position, the user can easily recognize the direction in which the container 200 should be moved according to the display of the indication display units 264a to 264d. Therefore, it is possible to quickly and efficiently activate the capsule endoscope 10.

### (Embodiment 3-1)

Next, Embodiment 3-1 of the present invention will be described.

FIG. 20 is a schematic diagram illustrating a configuration example of an activation system including an activation device according to Embodiment 3-1. The activation system 3-1 illustrated in FIG. 20 includes a capsule endoscope 10, a container 100 that houses the capsule endoscope 10, and an activation device 310 that activates the capsule endoscope 10. In FIG. 20, a receiving coil 18a is schematically illustrated and a cross-section is illustrated only for the container 100. The internal configuration of the activation device 310 is illustrated by a block diagram.

The activation device 310 includes a case 311. The upper surface of the case 311 has a planar shape and the upper surface is a placement surface 310a of the container 100. A guide display portion 312 that indicates a specific position on which the container 100 should be placed may be provided on the placement surface 310a.

The activation device 310 includes an inductance measurement unit 313 and a control unit 314 instead of the switch button 113 in the circuit configuration (see FIG. 6) included in the activation device 110 according to Embodiment 1-1. In FIG. 20, the capacitor 115 (see FIG. 6) that forms a resonance circuit with the transmitting coil 114 is omitted.

The inductance measurement unit 313 is connected to the transmitting coil 114 and measures an inductance of the transmitting coil 114 when the container 100 is placed on the placement surface 310a.

The control unit 314 estimates an electromotive force of the receiving coil 18a by calculating a mutual inductance with the receiving coil 18a based on a measurement result of the inductance measurement unit 313. Then, the control unit 314 determines a positional relationship between the receiving coil 18a and the transmitting coil 114 based on the estimation result.

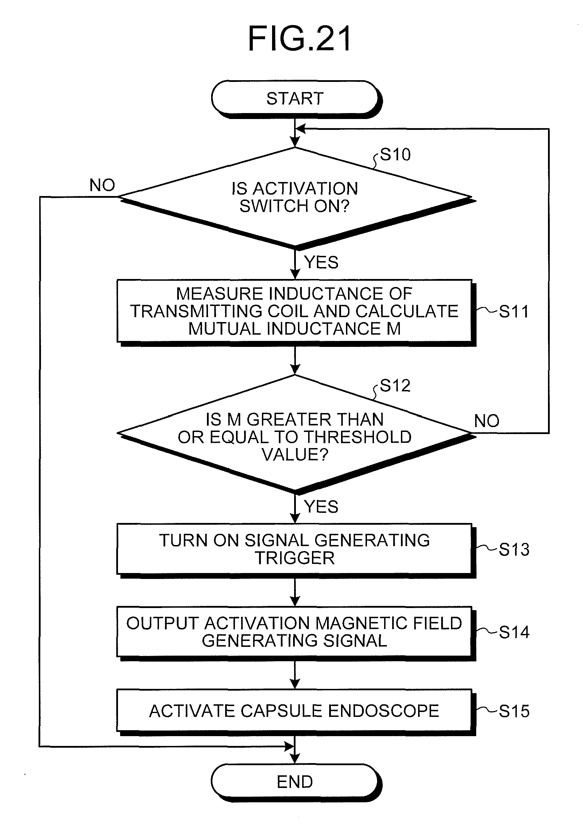

Next, the activation method of the capsule endoscope 10 will be described. FIG. 21 is a flowchart illustrating an operation of the activation system 3-1.

First, a user places the container 100 that houses the capsule endoscope 10 on the placement surface 310a of the activation device 310.

In step S10, the activation device 310 determines whether or not an activation switch (not shown in the drawings) is turned ON. When the activation switch is not turned ON (step S10: No), the operation of the activation device 310 directly ends.

When the activation switch is turned ON (step S10: Yes), the control unit 314 causes the power supply 116 to supply a weak test current to the transmitting coil 114 and calculates a mutual inductance M with the receiving coil 18a based on the inductance of the transmitting coil 114 measured by the inductance measurement unit 313 (step S11).

In the subsequent step S12, the control unit 314 determines whether or not the mutual inductance M is greater than or equal to a specified threshold value. The threshold value used at this time is set to a value where the receiving coil 18a can generate an electromotive force sufficient to turn the switch unit 18 (see FIG. 5) to ON state by an AC magnetic field generated by the transmitting coil 114.

When the mutual inductance M is greater than or equal to the specified threshold value (step S12: Yes), the control unit 314 turns ON a signal generating trigger for the transmitting coil 114 (step S13).

In step S14, the signal generator 117 outputs a signal (activation magnetic field generating signal) to cause the transmitting coil 114 to generate an AC magnetic field that can activate the capsule endoscope 10. The activation magnetic field generating signal may be a steady-state value or may be a signal having a specified pattern.

In step S15, an activation magnetic field is generated from the transmitting coil 114 and thereby an induced current or an induced voltage greater than or equal to a specified value is generated in the receiving coil 18a and the capsule endoscope 10 is activated. Alternatively, it may be configured so that the capsule endoscope 10 is activated when an induced current or an induced voltage having a specified pattern is generated in the receiving coil 18a.

Thereafter, the operation of the activation device 310 ends.

On the other hand, when the mutual inductance M is smaller than the specified threshold value (step S12: No), the operation of the activation device 310 proceeds to step S10. In this case, the user may adjust the position of the container 100 and search for the specific position where the capsule endoscope 10 is activated.

As described above, according to Embodiment 3-1, it is possible to directly detect whether or not the receiving coil 18a included in the capsule endoscope 10 is located at a position where the receiving coil 18a can be activated by the activation device 310 by generating a test magnetic field from the activation device 310. Therefore, it is possible to more reliably activate the capsule endoscope 10 and suppress the power consumption in the activation device 310.

Further, in this case, it is not necessary to provide members such as the portions to be detected to the container 100, so that the configuration of the container 100 can be simplified.

### (Modified Example 3-1-1)

Next, Modified Example 3-1-1 of Embodiment 3-1 of the present invention will be described.

There is a capsule endoscope 10 which includes a permanent magnet to control position and orientation when being inserted into a subject. When using such a capsule endoscope 10, a magnetic sensor is provided instead of the inductance measurement unit 313 described above, and whether or not the capsule endoscope 10 is located at a position where the capsule endoscope 10 can be activated by the activation device 310 may be determined by causing the magnetic sensor to detect a magnetic field formed by the permanent magnet in the capsule endoscope 10.

### (Modified Example 3-1-2)

Next, Modified Example 3-1-2 of Embodiment 3-1 of the present invention will be described.

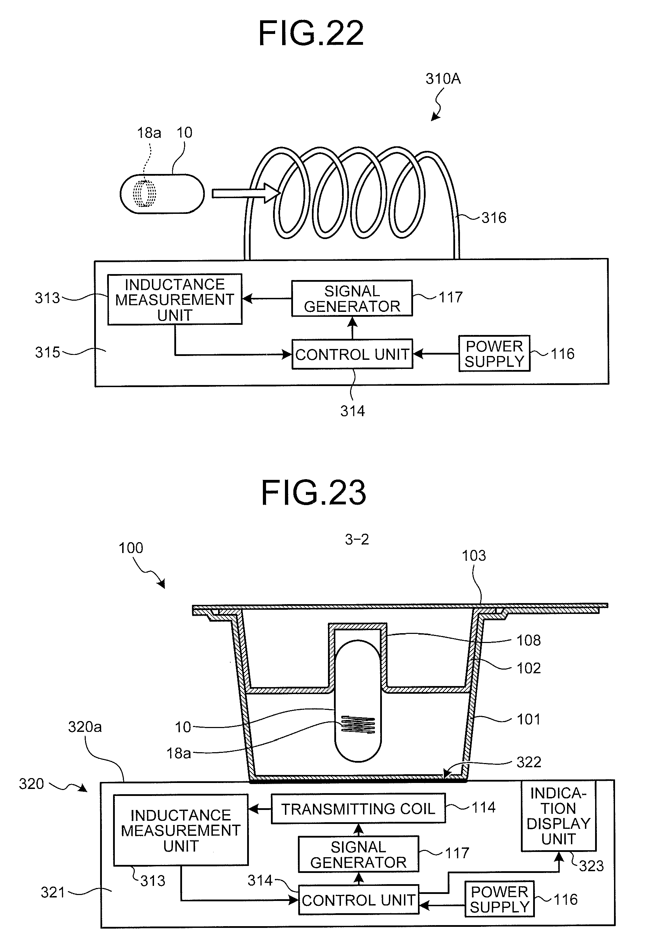

FIG. 22 is a schematic diagram illustrating a configuration example of an activation system including an activation device according to Modified Example 3-1-2. Embodiment 3-1 can be applied to an activation device 310A of a method in which the capsule endoscope 10 is activated by causing the capsule endoscope 10 to pass through an opening of a coil as illustrated in FIG. 22.

The activation device 310A illustrated in FIG. 22 includes a transmitting coil 316 provided on a case 315 instead of the transmitting coil 114 illustrated in FIG. 20. The configuration and the operation of each unit in the activation device 310A other than the transmitting coil 316 are the same as those in Embodiment 3-1.

When activating the capsule endoscope 10, the capsule endoscope 10 is inserted into the opening of the transmitting coil 316 in a state in which a weak current is applied to the transmitting coil 316. During the above operation, the inductance measurement unit 313 measures an inductance of the transmitting coil 316, and the control unit 314 calculates a mutual inductance M with the receiving coil 18a based on the measurement result. When the mutual inductance M becomes greater than or equal to a specified threshold value, the control unit 314 turns on a signal generating trigger for the transmitting coil 316 and causes the signal generator 117 to output an activation magnetic field generating signal. Thereby, an activation magnetic field is generated from the transmitting coil 316 and the capsule endoscope 10 is activated. The activation magnetic field generating signal may be a steady-state value or may be a signal having a specified pattern. Alternatively, it may be configured so that the capsule endoscope 10 is activated when current or voltage greater than or equal to a specified value is generated in the receiving coil 18a or the capsule endoscope 10 is activated when current or voltage having a specified pattern is generated in the receiving coil 18a.

As described above, according to Modified Example 3-1-2, when the capsule endoscope 10 is arranged at a position where the capsule endoscope 10 can be activated by a magnetic field generated by the transmitting coil 316, the activation magnetic field generating signal is outputted and a necessary current is applied to the transmitting coil 316, so that it is possible to suppress the power consumption of the activation device 310A.

### (Embodiment 3-2)

Next, Embodiment 3-2 of the present invention will be described.

FIG. 23 is a schematic diagram illustrating a configuration example of an activation system including an activation device according to Embodiment 3-2. The activation system 3-2 illustrated in FIG. 23 includes a capsule endoscope 10, a container 100 that houses the capsule endoscope 10, and an activation device 320 that activates the capsule endoscope 10. In FIG. 23, a receiving coil 18a is schematically illustrated and a cross-section is illustrated only for the container 100. The internal configuration of the activation device 320 is illustrated by a block diagram.

### The activation device 320 includes a case 321. The upper surface of the case 321 has a planar shape and the upper surface is a placement surface 320a of the container 100. A guide display portion 322 that indicates a specific position on which the container 100 should be placed may be provided on the placement surface 320a.

The activation device 320 further includes an indication display unit 323 that operates under control of the control unit 314 as compared with the activation device 310 illustrated in FIG. 20. The configuration and the operation of each unit in the activation device 320 other than the indication display unit 323 are the same as those in the in Embodiment 3-1.

The indication display unit 323 is a notification unit that notifies a user that the placement position of the container 100 is not appropriate when the container 100 on the placement surface 320a is shifted from the specific position, and the indication display unit 323 is also a guidance unit for guiding the container 100 to the specific position. The indication display unit 323 includes, for example, a light-emitting element such as an LED and is configured to light up with a plurality of colors (for example, red, yellow, green, and blue). Alternatively, the indication display unit 323 may be formed by a display panel or the like that can display characters.

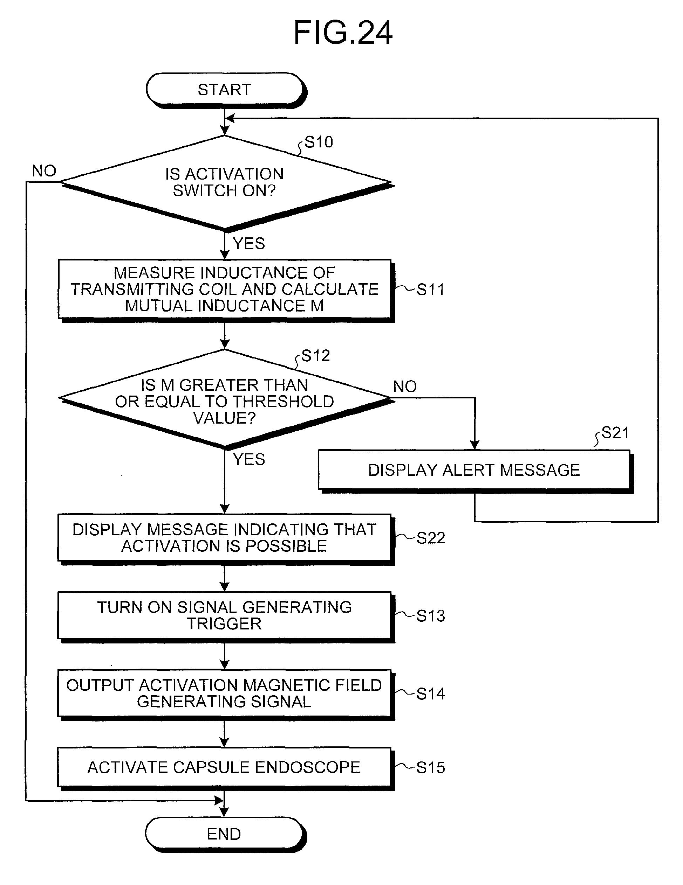

Next, the activation method of the capsule endoscope 10 will be described. FIG. 24 is a flowchart illustrating an operation of the activation system 3-2.

First, a user places the container 100 that houses the capsule endoscope 10 on the placement surface 320a of the activation device 320. The subsequent steps S10 to S12 are the same as those in Embodiment 3-1 (see FIG. 21).

In step S12, when the mutual inductance M is smaller than the specified threshold value (step S12: No), the control unit 314 displays an alert message to alert a user that the capsule endoscope 10 cannot be activated (step S21). In this case, the display method of the indication display unit 323 may be changed according to the state of the capsule endoscope 10. For example, when the capsule endoscope 10 cannot be activated at all (when a difference between the mutual inductance M and the threshold value is greater than a specified value), the indication display unit 323 is lit up with red color. When the capsule endoscope 10 approaches an area, where the capsule endoscope 10 can be activated, but cannot be reliably activated (when the mutual inductance M is smaller than the threshold value and the difference between the mutual inductance M and the threshold value is smaller than or equal to the specified value), the indication display unit 323 is lit up with yellow color. Alternatively, when the indication display unit 323 is formed by a display panel, a message indicating that "Activation is not possible, move the container to the center." may be displayed on the display panel.

On the other hand, when the mutual inductance M is greater than or equal to the specified threshold value (step S12: Yes), the control unit 314 displays a message indicating that the capsule endoscope 10 can be activated (step S22). Specifically, the indication display unit 323 may be lit up with green color, or when the indication display unit 323 is formed by a display panel, a message indicating that "Activation is possible" may be displayed on the display panel.

The subsequent steps S13 to S15 are the same as those in Embodiment 3-1. In step S15, when the capsule endoscope 10 is actually activated, the fact that the capsule endoscope 10 is activated may be notified to the user. Specifically, the indication display unit 323 may be lit up with blue color or a message "Capsule endoscope is activated" may be displayed on the display panel.

As described above, according to Embodiment 3-2, the user can easily recognize the position of the container 100 where the capsule endoscope 10 can be activated and reliably activate the capsule endoscope 10 by referring to the indication display unit 323.

### (Modified Example 3-2)

Next, Modified Example 3-2 of Embodiment 3-2 of the present invention will be described.

The switch button (see FIG. 1) is provided to the activation device 320 in the same manner as in Embodiment 1-1 and a user may manually give a signal generating trigger of step S13 by pressing the switch button. In this case, after the user places the container 100 at a position where the capsule endoscope 10 can be activated, the user can activate the capsule endoscope 10 at desired timing.

### (Embodiment 3-3)

Next, Embodiment 3-3 of the present invention will be described.

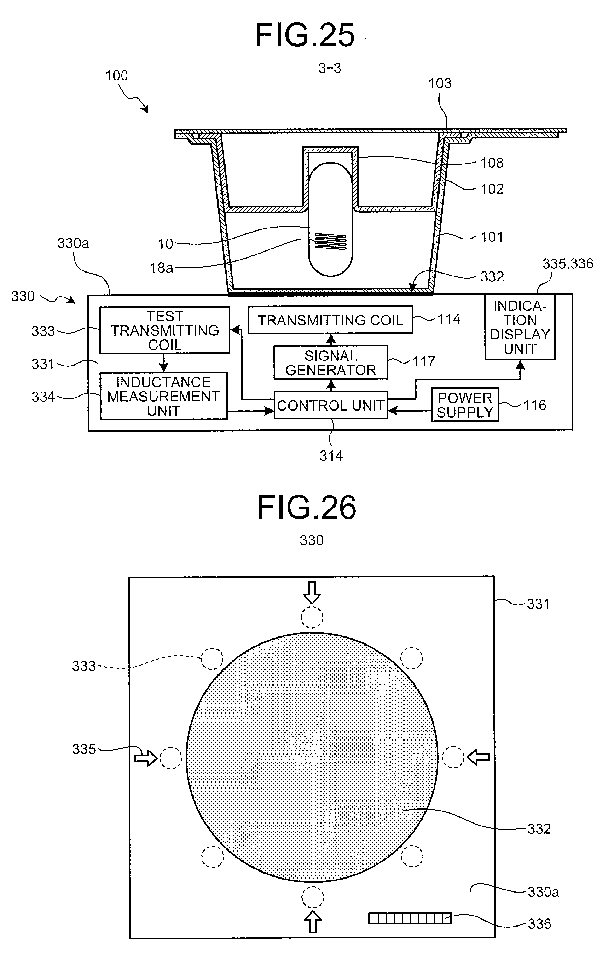

FIG. 25 is a schematic diagram illustrating a configuration example of an activation system including an activation device according to Embodiment 3-3. The activation system 3-3 illustrated in FIG. 25 includes a capsule endoscope 10, a container 100 that houses the capsule endoscope 10, and an activation device 330 that activates the capsule endoscope 10. In FIG. 25, a receiving coil 18a is schematically illustrated and a cross-section is illustrated only for the container 100. The internal configuration of the activation device 330 is illustrated by a block diagram.

The activation device 330 includes a case 331. The upper surface of the case 331 has a planar shape and the upper surface is a placement surface 330a of the container 100. A guide display portion 332 that indicates a specific position on which the container 100 should be placed may be provided on the placement surface 330a.

The activation device 330 further includes a plurality of test transmitting coils 333 as compared with the activation device 320 illustrated in FIG. 23. Further, the activation device 330 includes a plurality of inductance measurement units 334 and a plurality of indication display units 335 and 336 instead of the inductance measurement unit 313 and the indication display unit 323 illustrated in FIG. 23.

FIG. 26 is a top view illustrating the placement surface of the activation device 330. As illustrated in FIG. 26, the plurality of test transmitting coils 333 are provided at a plurality of positions near the placement surface 330a so that the opening surface is in parallel with the placement surface 330a. In Embodiment 3-3, the test transmitting coils 333 are arranged at eight positions near the circumference of the guide display portion 332.

The plurality of inductance measurement units 334 are connected to the test transmitting coils 333 respectively. Each inductance measurement unit 334 measures an inductance of the test transmitting coil 333 connected to the inductance measurement unit 334 when the container 100 is placed on the placement surface 330a.

The plurality of indication display units 335 and 336 are notification units that notify a user that the placement position of the container 100 is not appropriate when the container 100 on the placement surface 330a is shifted from the specific position, and the indication display units 335 and 336 are also guidance units for guiding the container 100 to the specific position. For example, the indication display units 335 and 336 include a light-emitting element such as an LED. Among them, the indication display units 335 are arrow-shaped areas provided at four positions around the guide display portion 332 and have a configuration to be able to light up with a plurality of colors (for example, red and green). On the other hand, the indication display unit 336 is an indicator provided at an edge portion of the placement surface 330a.

Next, the activation method of the capsule endoscope 10 will be described with reference to FIG. 24.

When the activation switch (not shown in the drawings) of the activation device 330 is turned on in step S10 (step S10: Yes), the control unit 314 causes the power supply 116 to supply a weak test current to each test transmitting coil 333 and calculates a mutual inductance M at the receiving coil 18a based on the inductances of the test transmitting coils 333 measured by each inductance measurement unit 334 (step S11).

In the subsequent step S12, the control unit 314 determines whether or not the mutual inductance M is greater than or equal to a specified threshold value. The threshold value used at this time is set to a value where the receiving coil 18a can generate an electromotive force sufficient to turn the switch unit 18 (see FIG. 5) to ON state by an AC magnetic field generated by the transmitting coil 114.

When the mutual inductance M is smaller than the specified threshold value (step S12: No), the control unit 314 displays an alert message to alert a user that the capsule endoscope 10 cannot be activated (step S21). Specifically, the control unit 314 estimates the position of the capsule endoscope 10 based on the measurement results of each inductance measurement unit 334, causes the indication display units 335 to display a direction in which the container 100 that houses the capsule endoscope 10 should be moved, and causes the indication display unit 336 to display an amount of movement by which the container 100 is moved.

On the other hand, when the mutual inductance M is greater than or equal to the specified threshold value (step S12: Yes), the control unit 314 displays a message indicating that the capsule endoscope 10 can be activated (step S22). Specifically, the control unit 314 lights up all of the four indication display units 335 or lights up the indication display units 335 with a color (for example, green) different from that in step S21. The operations in the subsequent steps S13 to S15 are the same as those in Embodiment 3-1.

As described above, according to Embodiment 3-3, it is possible to accurately grasp the position of the capsule endoscope 10 by providing a plurality of test transmitting coils 333, so that it is possible to display a more appropriate indication to a user with respect to the position where the container 100 should be placed. Therefore, the user can more reliably and efficiently activate the capsule endoscope 10.

### (Modified Examples)

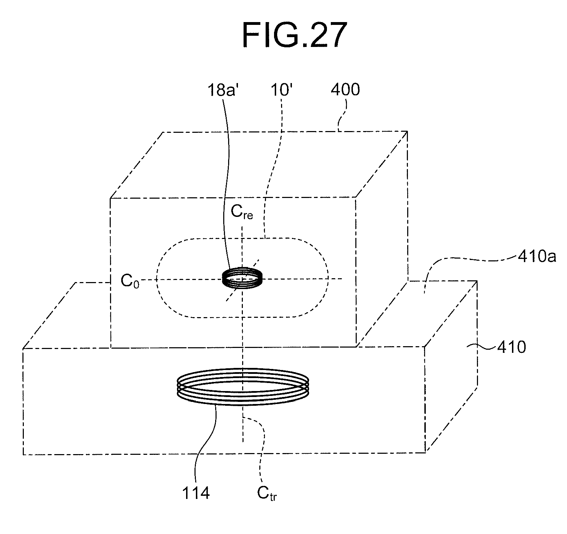

In Embodiments 1-1 to 1-3 described above, a case is described in which the central axis Cᵣₑ of the receiving coil 18a coincides with the central axis C₀ of the capsule endoscope 10. However, the arrangement of the coils in the capsule endoscope and the activation device is not limited if it is possible to cause the axis of the receiving coil in the capsule endoscope and the axis of the transmitting coil in the activation device to coincide with each other and it is possible to cause the openings thereof to face each other with a specified distance in between. For example, like a capsule endoscope 10' illustrated in FIG. 27, when a receiving coil 18a' is provided such that the central axis Cᵣₑ is perpendicular to the central axis C₀ of the case, the capsule endoscope 10' is held such that the central axis C₀ of the capsule endoscope 10' is in parallel with the bottom surface of a container 400. The container 400 is placed on a placement surface 410a of an activation device 410 including a transmitting coil 114 such that the placement surface 410a and the central axis Cᵣₑ of the receiving coil 18a' are perpendicular to each other. Thereby, a positional relationship in which the central axis Cᵣₑ of the receiving coil 18a' and the central axis Cₜᵣ of the transmitting coil 114 are substantially coincident with each other is achieved, so that it is possible to activate the capsule endoscope 10'.

In Embodiments 1-1 to 3-3 described above, an example is described in which the present invention is applied to an activation device and an activation system. However, the present invention may be applied as a control device including control of activation and/or stop of the capsule endoscope 10.

The present invention described above is not limited to Embodiments 1-1 to 3-3 and the modified examples thereof, but various inventions can be formed by appropriately combining a plurality of elements disclosed in the embodiments and the modified examples. For example, the inventions may be formed by removing some elements from all the elements described in each of the embodiments and the modified examples or may be formed by appropriately combining elements described in different embodiments and modified examples.

### Reference Signs List

- 1-1 to 1-5, 2-1 to 2-4, 3-1 to 3-3: activation system
- 10: capsule endoscope
- 11: closed container
- 11a: tip cover
- 11b: body portion cover
- 12a: LED drive circuit
- 13: CCD
- 13a: CCD drive circuit
- 14: image forming lens
- 15: RF transmitting unit
- 16: transmitting antenna unit
- 17: control unit
- 17a: system control unit
- 18: switch unit
- 18a: receiving coil
- 18b: capacitor
- 18c: diode
- 18d: smoothing capacitor
- 18e: resistor
- 18f: frequency dividing circuit
- 18g: power supply switch
- 19: battery
- 100, 140, 200, 220, 240, 400: container
- 101: outer container
- 102: inner lid portion
- 103, 143: outer lid portion
- 104: housing portion
- 105: handle portion
- 106: cylinder portion
- 107: engagement portion
- 108: holding portion
- 109: cutout
- 110, 120, 130, 150, 160, 210, 230, 250, 260, 310, 310A, 320, 330, 410: activation device
- 110a, 150a, 210a, 260a, 310a, 320a, 330a, 410a: placement surface
- 111, 121, 131, 211, 221, 251, 261, 311, 315, 321, 331: case
- 112: guide display portion
- 113: switch button
- 114, 316: transmitting coil
- 115: capacitor
- 116: power supply
- 117: signal generator
- 117a: oscillator
- 117b: timing generator
- 117c: driver
- 118: weight sensor
- 119: switch unit
- 122: convex portion
- 123, 133: placement portion
- 132: concave portion
- 141: container main body
- 142: inner container
- 144: holding portion
- 151, 231: pedestal
- 152, 232: wall portion
- 153, 154, 234: abutting surface
- 201: portion to be detected
- 221, 241: container main body
- 222, 242: side surface
- 212: guide display portion
- 213, 235, 255, 256: detection unit
- 214: switch unit
- 233: abutting member
- 265, 314: control unit
- 253, 254: guide wall
- 252: side surface
- 262, 312, 322, 332: guide display portion
- 263a to 263d: detection unit
- 264a to 264d, 323, 335, 336: indication display unit
- 313,: 334 inductance measurement unit
- 333: test transmitting coil

## Claims

1. An activation device for activating a capsule medical device that has a first coil inside and is activated when voltage greater than or equal to a specified value or current greater than or equal to a specified value is generated in the first coil, the activation device comprising:
a case;
a second coil that is provided in the case and is configured to generate a magnetic field when current flows through the second coil; and
a guide portion for guiding to a position that is located outside the case and that is a position of the capsule medical device where the capsule medical device can be activated based on the magnetic field generated by the second coil.

2. The activation device according to claim 1, wherein when the second coil generates a magnetic field with a predefined pattern, and voltage or current having a predefined pattern is generated in the first coil, the capsule medical device is activated.

3. The activation device according to claim 1 or 2, wherein
the capsule medical device is housed at a specified position in a specified posture in a container having a specified shape,
the case has a placement surface on which the container is configured to be placed, and
the guide portion is configured to guide to a specific position on the placement surface where the capsule medical device can be activated when the container is placed on the placement surface.

4. The activation device according to claim 3, wherein the guide portion is display indicating the specific position.

5. The activation device according to claim 3, wherein the guide portion is a concave portion or a convex portion provided at the specific position.

6. The activation device according to claim 3, wherein the guide portion is a member provided adjacent to the specific position and having an abutting surface to abut against a part of the container.

7. The activation device according to any one of claims 3 to 6, further comprising:
a detection unit configured to detect that the container is placed at the specific position and to output a detection signal; and
a control unit configured to cause current to be applied to the second coil when the detection unit outputs the detection signal.

8. The activation device according to claim 1 or 2, wherein
the capsule medical device is housed at a specified position in a specified posture in a container having a specified shape,
the case has a placement surface on which the container is configured to be placed, and
the guide portion includes:
a detection unit configured to detect a position of the capsule medical device placed on the placement surface and to output a signal indicating the position; and
a guidance unit configured to guide the container to a specific position on the placement surface where the capsule medical device can be activated when the container is placed on the placement surface, based on the signal.

9. The activation device according to claim 8, wherein the guidance unit includes a light-emitting element provided on the placement surface.

10. The activation device according to claim 8 or 9, wherein
the container is provided with a portion to be detected that can be detected by the detection unit, and
the detection unit outputs the signal when detecting the portion to be detected.

11. The activation device according to any one of claims 8 to 10, further comprising a control unit configured to cause current to be applied to the second coil based on the signal outputted by the detection unit.

12. The activation device according to claim 1 or 2, wherein the guide portion includes:
a signal generator configured to generate a signal to apply a test current to the second coil;
a magnetic field detection unit configured to detect a change in magnetic field outside the case; and
a control unit configured to cause the current applied to the second coil to increase when the magnetic field detection unit detects the change in magnetic field greater than or equal to a specified value.

13. The activation device according to claim 12, wherein the guide portion further includes an indication display unit configured to perform a specified display according to the change in magnetic field detected by the magnetic field detection unit.

14. The activation device according to claim 13, wherein the indication display unit is at least one of a light-emitting element, an indicator, and a display panel, which are provided on the placement surface.
